(19) Europäisches Patentamt
European Patent Office
Office européen des brevets

(11) Veröffentlichungsnummer: 0 111 442
A1

(12) # EUROPÄISCHE PATENTANMELDUNG

(21) Anmeldenummer: 83810511.2

(22) Anmeldetag: 07.11.83

(51) Int. Cl.[3]: C 07 D 413/12
C 07 D 409/12, C 07 D 403/12
A 01 N 47/36

(30) Priorität: 12.11.82 CH 6612/82
29.03.83 CH 1722/83

(43) Veröffentlichungstag der Anmeldung: 20.06.84 Patentblatt 84/25

(84) Benannte Vertragsstaaten: BE CH DE FR GB IT LI NL

(71) Anmelder: CIBA-GEIGY AG
Postfach
CH-4002 Basel(CH)

(72) Erfinder: Pissiotas, Georg, Dr.
Breslauerstrasse 8
D-7850 Lörrach(DE)

(72) Erfinder: Schurter, Rolf, Dr.
Holzmattstrasse 45
D-4102 Binningen(CH)

(54) **N-2-Heterocyclyl-phenylsulfonyl-N'-pyrimidinyl- und -triazinyl-harnstoffe.**

(57) N-2-Heterocyclylphenylsulfonyl-N'-pyrimidinyl- und -triazinyl-harnstoffe der allgemeinen Formel

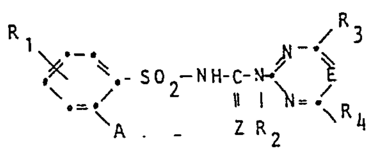

und die Salze dieser Verbindungen mit Aminen, Alkali- oder Erdalkalimetallbasen oder mit quaternären Ammoniumbasen haben gute pre- und postemergent-selektive herbizide und wuchsregulierende Eigenschaften.

In dieser Formel bedeuten Z Sauerstoff oder Schwefel; E Stickstoff oder =CH−; $R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy; $R_2$ Wasserstoff, $C_1$-$C_4$-Alkyl oder $C_1$-$C_3$-Alkoxy; $R_3$ und $R_4$ unabhängig voneinander Wasserstoff, $C_1$-$C_4$-Alkyl, $C_1$-$C_4$-Haloalkyl, $C_3$-$C_5$-Cycloalkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkoxy oder $-NR_5R_6$, wobei $R_5$ und $R_6$ für Wasserstoff oder $C_1$-$C_4$-Alkyl stehen, und A einen über Kohlenstoff gebundenen, ungesättigten oder nur teilweise gesättigten 5-6 gliedrigen Heterocyclus, der 1, 2 oder 3 Heteroatome enthält und durch $C_1$-$C_4$-Alkyl, $C_1$-$C_4$-Halogenalkyl, $C_1$-$C_4$-Alkoxy, $C_1$-$C_4$-Alkylthio, $C_2$-$C_8$-Alkoxyalkyl, Di($C_1$-$C_4$Alkyl)amino, Halogen, Cyan oder Nitro substituiert sein kann, bedeuten.

EP 0 111 442 A1

CIBA-GEIGY AG 5-14180/1+2/+

Basel (Schweiz)

N-2-Heterocyclyl-phenylsulfonyl-N'-pyrimidinyl- und -triazinyl-harnstoffe.

Die vorliegende Erfindung betrifft neue, herbizid wirksame und pflanzenwuchsregulierende N-2-Heterocyclyl-phenylsulfonyl-N'-pyrimidinyl- und -triazinyl-harnstoffe, Verfahren zu ihrer Herstellung, sie als Wirkstoffe enthaltenden Mittel sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue 2-Heterocyclyl-phenylsulfonylisocyante, -thioisocyanate-, -phenylsulfonylcarbamate sowie 2-Heterocyclylphenylsulfonamide.

Die erfindungsgemässen N-2-Heterocyclyl-phenylsulfonyl-N'-pyrimidinyl- und -triazinyl-harnstoffe entsprechen der allgemeinen Formel I

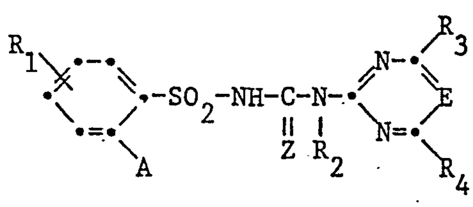

worin

Z Sauerstoff oder Schwefel,

E Stickstoff oder =CH-,

$R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R_2$ Wasserstoff, $C_1$-$C_4$-Alkyl oder $C_1$-$C_3$-Alkoxy,

$R_3$ und $R_4$ unabhängig voneinander Wasserstoff, $C_1$-$C_4$-Alkyl, $C_1$-$C_4$-Halogenalkyl, $C_3$-$C_5$-Cycloalkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkyl,

$C_2$-$C_5$-Alkoxyalkoxy oder $-NR_5R_6$, wobei $R_5$ und $R_6$ für Wasserstoff oder $C_1$-$C_4$-Alkyl stehen, und A einen über Kohlenstoff gebundenen ungesättigten oder nur teilweise gesättigten, 5-6 gliedrigen Heterocyclus, welcher 1, 2 oder 3 Heteroatome enthält und durch $C_1$-$C_4$-Alkyl, $C_1$-$C_4$-Halogenalkyl, $C_1$-$C_4$-Alkoxy, $C_1$-$C_4$-Alkylthio, $C_2$-$C_8$-Alkoxyalkyl, Di($C_1$-$C_4$-Alkyl)amino, Halogen, Cyan oder Nitro substituiert sein kann, bedeuten, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise im niederländischen Patent Nr. 121 788, im US-Patent Nr. 4 127 405 und in den europäischen Patentanmeldungen 44807, 44808 und 51465 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl. Cycloalkylreste sind z.B. Cyclopropyl, Cyclobutyl und Cyclopentyl, die auch durch Methyl substituiert sein können.

Unter Alkoxy ist zu verstehen: Methoxy, Aethoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Aethoxy oder i-Propyloxy.

Beispiele für Alkylthio sind Methylthio, Aethylthio, n-Propylthio, i-Propylthio, n-Butylthio oder n-Pentylthio insbesondere aber Methylthio und Aethylthio.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl oder n-Propylsulfonyl, insbesondere aber Methylsulfonyl und Aethylsulfonyl.

Unter Halogen in den Definitionen sowie als Teil in Halogenalkoxy sind Fluor, Chlor und Brom, vorzugsweise jedoch Fluor und Chlor zu verstehen.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Als ungesättigte oder nur teilweise gesättigte, 5-6 gliedrige Heterocyclen A, welche 1-3 Heteroatome enthalten kommen in Frage:
Thiophen, Furan, Pyrrol, Isothiazol, Thiazol, Isoxazol, Oxadiazole, Oxazol, Pyrazol, Imidazol, Pyran, Pyridin, Pyrazin, Pyrimidin, Pyridazin, 1,2-Oxazin, 1,3-Oxazin, 1,2-Thiazin, 1,3-Thiazin, 1,4-Thiazin, 1,2,3-Triazol, 1,2,4-Triazol, 1,2,3-Thiadiazin, 1,2,4-Thiadiazin, 1,3,4-Thiadiazin, Furazan, Pyrroline, Imidazolin, Pyrazolin, Oxazolin

Thiazolidine, Thiazoline, 1,3,5-Triazine oder 1,3,4-Triazine. Diese Heterocyclen können ein- bis dreifach durch $C_1$-$C_4$ Alkyl, $C_1$-$C_4$ Haloalkyl, $C_1$-$C_4$ Alkoxy, $C_1$-$C_4$ Haloalkoxy, $C_2$-$C_8$ Alkoxyalkoxy, $C_1$-$C_4$ Alkylthio, Mono- oder Di($C_1$-$C_4$ Alkyl)-amino, Halogen, Cyan oder Nitro substituiert sein.

Unter den Verbindungen der Formel I sind diejenigen bevorzugt, in denen entweder

- der Substituent Z Sauerstoff ist oder
- der Substituent $R_1$ Wasserstoff darstellt oder
- der Substituent $R_2$ Wasserstoff oder Methyl darstellt, oder
- A einen über Kohlenstoff gebundenen und durch $C_3$-$C_4$ Alkyl, $C_1$-$C_4$ Halogenalkyl, $C_3$-$C_4$ Alkoxy, $C_2$-$C_4$ Alkylthio, $C_2$-$C_8$ Alkoxyalkyl, Di($C_1$-$C_4$)alkylamino, Cyan oder Nitro substituierten gesättigten 5-6 gliedrigen Heterocyclus welcher 1, 2 oder 3 Heteroatome enthält, darstellt oder
- A den 5-Trifluormethyl-1,3,4-oxadiazol-2-yl- oder den 5-t.Butyl-1,3,4-oxadiazol-2-ylrest darstellen.
- ferner die Verbindungen

N-[2-(3-Methyl-1,2,4-oxadiazol-5-yl)-phenylsulfonyl]-N'-(4-cyclopropyl-6-methoxy-1,3,5-triazin-2-yl)harnstoff,

N-[2-(1,3,4-Oxadiazol-2-yl)-phenylsulfonyl]-N'-(4-difluormethoxy-6-methyl-pyrimidin-2-yl)-harnstoff,

N-[2-(1,2-Oxazol-5-yl)-phenylsulfonyl]-N'-(4-difluormethoxy-6-methyl-pyrimidin-2-yl,

N-[2-(5-Methyl-1,3,4-oxadiazol-2-yl)-phenylsulfonyl]-N'-(4-difluormethoxy-6-methyl-pyrimidin-2-yl)harnstoff,

N-[2-(5-t-Butyl-1,3,4-oxadiazol-2-yl)-phenylsulfonyl]-N'-(4-methoxy-6-methylpyrimidin-2-yl)harnstoff,

N-[2-(5-Trifluormethyl-1,3,4-oxadiazol-2-yl)-phenylsulfonyl]-N'-(4-methoxy-6-methylpyrimidin-2-yl)harnstoff.

- Schliesslich herbizide Mittel zur selektiven Unkrautbekämpfung in Nutzpflanzenkulturen wie beispielsweise Soja und Reis welche als Wirkstoff

N-[2-(3-Methyl-1,2,4-oxadiazol-5-yl)phenylsulfonyl]-N'-(4-chlor-6-methoxy-pyrimidin-2-yl)harnstoff,

N-[2-(Thiophen-2-yl)-phenylsulfonyl]-N'-(4,6-dimethoxy-pyrimidin-2-yl)-harnstoff,

N-[2-(1,3,4-Oxadiazol-2-yl)-phenylsulfonyl]-N'-(4-chlor-6-methoxy-pyrimidin-2-yl)harnstoff enthält.

Die Herstellung der Verbindungen der Formel I erfolgt in einem inerten, organischen Lösungsmittel.

Nach einem ersten Verfahren werden die Verbindungen der Formel I erhalten, indem man ein 2-Heterocyclyl-phenylsulfonamid der Formel II

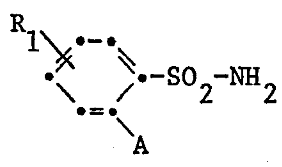 (II),

worin $R_1$ und A die unter Formel I gegebene Bedeutung haben, gegebenenfalls in Gegenwart einer Base mit einem N-Pyrimidinyl- oder -triazinylcarbamat der Formel III

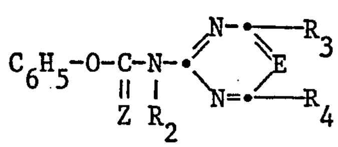 (III),

worin

E, $R_2$, $R_3$, $R_4$ und Z die unter Formel I gegebene Bedeutung haben, umsetzt.

Nach einem zweiten Verfahren gelangt man zu Verbindungen der Formel I, indem man ein 2-Heterocyclyl-phenylsulfonylisocyanat oder -isothiocyanat der Formel IV

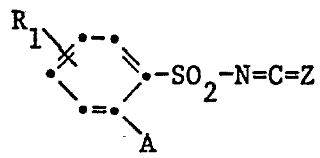 (IV),

worin A, $R_1$ und Z die unter Formel I gegebene Bedeutung haben, gegebenenfalls in Gegenwart einer Base mit einem Aminopyridin oder -triazin der Formel V

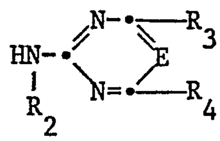 (V),

worin
E, $R_2$, $R_3$ und $R_4$ die unter Formel I gegebene Bedeutung haben, umsetzt.

Schliesslich kann man die Verbindungen der Formel I auch erhalten, indem man ein N-2-Heterocyclyl-phenylsulfonylcarbamat der Formel VI

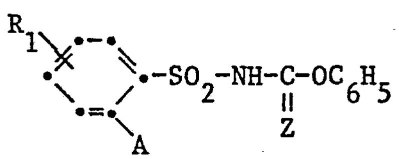 (VI),

worin A, $R_1$ und Z die unter Formel I gegebene Bedeutung haben, mit einem Aminopyrimidin oder -triazin der oben angegebene Formel V umsetzt.

Die erhaltene Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Ausgangsstoffe der Formeln II, IV und VI sind neu und können nach den folgenden Methoden hergestellt werden:

Die neuen und als Zwischenprodukte verwendeten substituierten Phenylsulfonamide der Formel II werden z.B. aus den entsprechenden Anilin-Derivaten durch Diazotierung und Austausch der Diazogruppe mit Schwefeldioxid in Gegenwart eines Katalysators wie Kupfer-I-chlorid in Salzsäure oder Essigsäure und Umsetzen des entstandenen Phenylsulfonylchlorids mit Ammoniak erhalten. Entsprechende Anilin-Derivate sind nach bekannten Methoden herstellbar. Die Phenylsulfonylchloride können auch durch oxydierende Chlorierung in essig- oder salzsaurem Medium aus den entsprechenden Thiophenolen, Diarylsulfiden und Thiophenolethern hergestellt werden. (S.F. Muth in Houben-Weyl's Methoden der Organischen Chemie, Bd. 9, 4. Aufl., G.Thieme Verlag, Stuttgart, 1955, S. 557ff.)

Die Zwischenprodukte der Formel II, IV, VI sind neu und speziell für die Synthese von Verbindungen der Formel I entwickelt worden. Sie bilden deshalb weitere Gegenstände der vorliegenden Erfindung.

Die substituierten Phenylsulfonylisocyanate der Formel IV können durch Umsetzung der Sulfonamide der Formel II mit Phosgen in Anwesenheit von Butylisocyanat in einem chlorierten Kohlenwasserstoff als Lösungsmittel bei Rückflusstemperatur erhalten werden. Aehnliche Herstellungsmethoden sind in "Neuere Methoden der präparativen organischen Chemie", Band VI, 211-229, Verlag Chemie, Weinheim, 1970, beschrieben.

Die substituierten Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 299, 174 (1966) beschrieben.

Die substituierten N-Phenylsulfonylcarbamate der Formel VI werden durch Umsetzung der Sulfonamide der Formel II mit Diphenylcarbonat oder Chlorameisensäurephenylester oder deren Schwefelanalogen in Gegenwart einer Base hergestellt. Aehnliche Verfahren sind in der japanischen Patentschrift 61169 erwähnt.

Die als Ausgangsmaterialien verwendeten Aminopyrimidine und -triazine der Formel V sowie entsprechende Phenylcarbamate der Formel III sind entweder seit langem bekannt oder in der schweizerischen Patentanmeldung Nr. 3527/82-8 beschrieben oder sie lassen sich nach bekannten Methoden aus dort beschriebenen Verbindungen erhalten.

Diese Umsetzungen zu Verbindungen der Formel I werden vorteilhafterweise in aprotischen, inerten, organischen Lösungsmitteln oder Lösungsmittelgemischen vorgenommen. Geeignete Lösungsmittel sind Aether wie Dioxan, Diäthyläther, Tetrahydrofuran, Aethylenglykoldimethyläther, Diäthylenglykoldimethyläther; Kohlenwasserstoffe wie Benzol, Toluol, Xylol, Cyclohexan; Nitrile wie Acetonitril, Propionitril; chlorierte Kohlenwasserstoffe wie Methylenchlorid, Chloroform, Tetrachlorkohlenstoff, Chlorbenzol oder Dimethylsulfoxid. Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit das Reaktionsgemisch aufgewärmt. Die Reaktionszeiten können ebenfalls durch Zugabe einiger Tropfen Base als Reaktionskatalysator verkürzt werden.

Als Basen können sowohl organische Basen wie Amine, wie Triäthylamin, Chinuclidin, Pyridin etc. als auch organische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohhlenwasserstoffen oder chlorierten

Kohlenwasserstoffen, gereinigt werden.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragsteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel,

Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbnezolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol oder Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Pulbikationen beschrieben:

"Mc Cutcheon's Detergents and Emulsifiers Annual" MC Publishing Corp., Ridgewood, New Jersey, 1979.

Sisley and Wood, "Encyclopedia of Surface Active Agents". Chemical Publishing Co., Inc. New York, 1964.

Die pestiziden Zubereitungen enthalten in der Regel 0,1 bis 95%, insbesondere 0.1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

<u>Emulgierbare Konzentrate</u>

| | |
|---|---|
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

<u>Stäube</u>

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

<u>Suspensions-Konzentrate</u>

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

<u>Benetzbare Pulver</u>

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0.001% an Wirkstoff verdünnt werden. Die Aufwandmengen betragen in der Regel 0,01 bis 10 kg AS/ha, vorzugsweise 0.025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung von N-2-(3-Methyl-1,2,4-oxadiazol-5-yl)-benzolsulfonyl-N'-(4-methoxy-6-methyl-pyrimidin-2-yl)-harnstoff

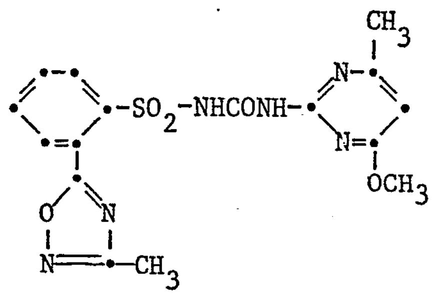

a) N-[(Dimethylamino)äthyliden]-2-benzylmercaptobenzamid

Ein Gemisch von 24,3 g 2-Benzylmercaptobenzamid und 50 ml N,N-Dimethylacetamid-dimethylacetal wird während 1 1/2 Stunden bei 120° gerührt und das dabei entstehende Methanol abdestilliert. Beim Abkühlen kristallisiert das N-[(Dimethylamino)-äthyliden]-2-benzylmercaptobenzamid. Man erhält 27 g (86,4% der Theorie) Amid vom Schmelzpunkt 142-143°.

b) 5-[2-Benzylmercaptophenyl)-3-methyl-1,2,4-oxadiazol

Ein Gemisch von 7,2 g Hydroxylamin-Hydrochlorid, 20,7 ml 5N Natronlauge und 150 ml 70%ige Essigsäure wird mit 27 g N-[(Dimethylamino)-äthyliden]-2-benzylmercaptobenzamid versetzt und 20 Minuten bei Raumtemperatur gerührt. Dann wird auf 5° abgekühlt und das Reaktionsgemisch mit 65 ml Wasser verdünnt. Es fällt ein Niederschlag aus, der abfiltriert und mit kaltem Wasser gewaschen wird. Nach Umkristallisation aus Acetonitril erhält man 15 g (61,7 % der Theorie) 5-[2-Benzylmercaptophenyl)-3-methyl-1,2,4-oxadiazol vom Schmelzpunkt 115°.

c) 2-(3-Methyl-1,2,4-oxadiazol-5-yl)-benzolsulfonsäurechlorid

In eine Suspension von 15 g 5-(2-Benzylmercaptophenyl)-3-methyl-1,2,4-oxadiazol in 60 ml Eisessig und 60 ml Wasser leitet man bei einer Temperatur von -5°-0° unter kräftigem Rühren so lange Chlor ein, bis eine Emulsion entstanden ist. Dann verdünnt man das Reaktionsgemisch mit der dreifachen Menge Eis/Wasser. Es bildet sich ein Niederschlag von dem man das Wasser vorsichtig abgiesst. Schliesslich versetzt man den Rückstand mit Petroläther filtriert und wäscht den Filterrückstand mit kaltem Wasser. Nach dem Trocknen verbleiben 12,9 g (92% der Theorie) 2-(3-Methyl-1,2,4-oxadiazol-5-yl)-benzolsulfonsäurechlorid, welches bei 72-73° schmilzt.

d) 2-(3-Methyl-1,2,4-oxadiazol-5-yl)-benzolsulfonamid

Zu einer gekühlten, mit Ammoniak übersättigten Tetrahydrofuran-Lösung gibt man unter Rühren 51,7 g 2-(3-Methyl-1,2,4-oxadiazol-5-yl)-benzolsulfonsäurechlorid. Nachdem alles zugegeben ist, rührt man eine Stunde bei Raumtemperatur und filtriert das ausgefallene Ammoniumchlorid ab. Die Tetrahydrofuran-Lösung wird eingedampft und zurück bleiben 44 g (92% der Theorie) 2-(3-Methyl-1,2,4-oxadiazol-5-yl)-benzolsulfonamid, welches bei 157-158° schmilzt.

Gemäss diesem Beispiel werden die in der Tabelle 1 aufgeführten Benzolsulfonamide hergestellt.

e) 2-(3-Methyl-1,2,4-oxadiazol-5-yl)-benzolsulfonyl-isocyanat

Zu einer Suspension von 35,9 g 2-(3-Methyl-1,2,4-oxadiazol-5-yl)-benzolsulfonamid in 400 ml Xylol gibt man 14,9 g n-Butylisocyanat. Anschliessend leitet man unter Rühren bei 140–145°C 59,4 g Phosgen ein. Nach Beendigung der Phosgenzugabe wird das überschüssige Phosgen mit Stickstoff entfernt und anschliessend das Lösungsmittel unter Vakuum abdestilliert. Als Rückstand verbleibt das Isocyanat als dickflüssiges Oel, das sofort weiter umgesetzt wird.

f) N-2-(3-Methyl-1,2,4-oxadiazol-5-yl)-benzolsulfonyl-N'-(4-methoxy-6-methyl-pyrimidin-2-yl)-harnstoff.

Ein Gemisch von 2 g 2-Amino-4-methoxy-6-methylpyrimidin und 3,9 g 2-(3-Methyl-1,2,4-oxadiazol-5-yl)-benzolsulfonylisocyanat in 70 ml Dioxan wird während 6 Stunden bei Rückfluss gerührt. Anschliessend wird das Lösungsmittel im Vakuum abdestilliert. Man erhält so 5,8 g (98 % der Theorie) N-[2-(3-Methyl-1,2,4-oxadiazol-5-yl)-benzolsulfonyl]-N'-(4-methoxy-6-methyl-pyrimidin-2-yl)-harnstoff vom Schmelzpunkt 195 - 196°.

<u>Beispiel 2:</u> Herstellung von N-2-(5-t.Butyl-1,3,4-oxadiazol-2-yl)-phenyl-N'-(4'-methoxy-6'-methyl-pyrimidin-2-yl)harnstoff

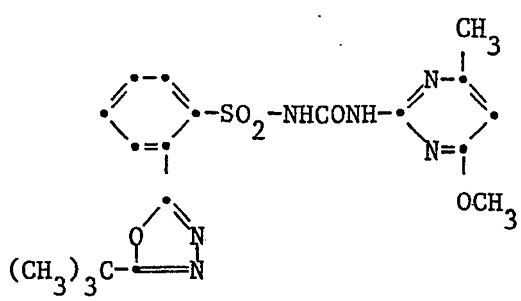

a) 2-Benzylmercaptobenzoylhydrazid
Ein Gemisch von 272 g 2-Benzyl-mercaptobenzoesäureester und 160 ml Hydrazinhydrat wird 4 Stunden bei einer Badtemperatur von 130° erhitzt und nach dem Abkühlen in Eiswasser gegossen. Die anfallenden weissen Kristalle werden getrocknet. Ausbeute 249 g Hydrazid. Schmelzpunkt 162-164°.

b) N-(2-Benzylmercaptobenzoyl)-N'-pivaloylhydrazin
Zu einer Suspension von 129 g 2-Benzylmercaptobenzoylhydrazid in 500 ml Toluol wird 101,5 ml Pivaloylsäurehydrazid derart zugetropft, dass die Reaktionstemperatur zwischen 30 und 40° bleibt. Nach der Zugabe wird die Reaktionsmischung 14 Stunden bei Raumtemperatur gerührt und dann eingedampft. Der Rückstand wird mit wenig Acetonitril gewaschen und anschliessend getrocknet. Ausbeute 160 g Pivaloylhydrazin, Schmelzpunkt 128 - 129°.

c) 2-(2-Benzylmercaptophenyl)-5-t.butyl-1,3,4-oxadiazol
Eine Mischung von 160 g N-2-(2-Benzylmercaptobenzoyl)-N'-pivaloylhydrazin und 400 g Phosphoroxychlorid wird 5 Stunden auf 120° erhitzt und nach dem Abkühlen vorsichtig ins Eiswasser gegossen. Die ausgefallene Substanz wird abgenutscht und aus Acetonitril/Wasser umkristallisiert Ausbeute 151 g Oxadiazol Schmelzpunkt 80 - 81°.

d) 2-(5-t.Butyl-1,3,4-oxadiazol-2-yl)-benzolsulfonsäurechlorid
In eine Suspension von 113,5 g 2-(2-Benzoylmercaptophenyl)-5-t.butyl-1,3,4-oxadiazol in 400 ml Eisessig und 400 ml Wasser wird bei -10° solange Chlor eingeleitet, bis das Ausgangsmaterial verbraucht ist. Dann wird das Reaktionsgemisch mit Eiswasser verdünnt, das ausgefallene Reaktionsprodukt abgenutscht und mit Hexan gewaschen. Ausbeute 88 g Sulfochlorid, Schmelzpunkt 107 - 108°.

e) N-2-(5-t.Butyl-1,3,4-oxadiazol-2-yl)-benzolsulfonamid
Zu einer gekühlten mit Ammoniak gesättigten Tetrahydrofuranlösung gibt man unter Rühren 81,5 g 2-(5-t.Butyl-1,3,4-oxadiazol-2-yl)-benzolsulfonsäurechlorid. Nachdem alles zugegeben ist, rührt man eine Stunde bei Raumtemperatur und filtriert das ausgefallene Ammoniumchlorid ab. Die Tetrahydrofuranlösung wird eingedampft, und aus dem Rückstand kristallisiert weisses Sulfonamid, Ausbeute 78 g Schmelzpunkt 170°.

f) 2-(5-t.Butyl-1,3,4-oxadiazol-2-yl)-benzolsulfonyl-isocyanat
Zu einer Suspension von 42 g 2-(5-t.Butyl-1,3,4-oxadiazol-2-yl)-benzolsulfonamid in 400 ml Xylol gibt man 14,9 g n-Butylisocyanat. Anschliessend leitet man unter Rühren bei 140-145°C 59,4 g Phosgen ein. Nach Beendigung der Phosgenzugabe wird das überschüssige Phosgen mit Stickstoff entfernt und anschliessend das Lösungsmittel unter Vakuum abdestilliert. Als Rückstand verbleibt das Isocyanat als dickflüssiges Oel, das sofort weiter umgesetzt wird.

g) N-2-(5-t.Butyl-1,3,4-oxadiazol-2-yl)benzolsulfonyl-N'-(4-methoxy-6-methyl-pyrimidin-2-yl)harnstoff.

Ein Gemisch von 2 g 2-Amino-4-methoxy-6-methylpyrimidin und 3,9 g 2-(5-Butyl-1,3,4-oxadiazol-2-yl)-benzolsulfonylisocyanat in 70 ml Dioxan wird während 6 Stunden bei Rückfluss gerührt. Anschliessend wird das Lösungsmittel im Vakuum abdestilliert. Man erhält so 5.6 g Titelprodukt, Schmelzpunkt 185°.

In analoger Weise zu diesem Beispiel werden die in der Tabelle 2 aufgeführten Sulfonylharnstoffe hergestellt.

Tabelle 1

$Q-SO_2NH_2$ = 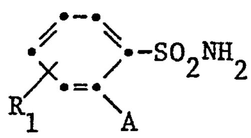

| Verbin-dung No. | Ring-system No. | |
|---|---|---|
| 1.1 | $Q_1$ | 2-(1,2,4-Oxadiazol-5-yl)benzol-sulfonamid |
| 1.2 | $Q_2$ | 2-(1,2,4-Oxadiazol-3-yl)benzol-sulfonamid |
| 1.3 | $Q_3$ | 2-(3-Methyl-1,2,4-oxadiazol-5-yl)benzol-sulfonamid |
| 1.4 | $Q_4$ | 2-(1,2,4-Thiadiazol-5-yl)benzol-sulfonamid |
| 1.5 | $Q_5$ | 2-(1,2,4-Thiadiazol-3-yl)phenyl-sulfonamid |
| 1.6 | $Q_6$ | 2-(1,3,4-Thiadiazol-2-yl)phenyl-sulfonamid |
| 1.7 | $Q_7$ | 2-(1-Methyl-1,2,4-triazol-5-yl)phenyl-sulfonamid |
| 1.8 | $Q_8$ | 2-(1-Methyl-1,2,4-triazol-3-yl)phenyl-sulfonamid |
| 1.9 | $Q_9$ | 2-(1,2,4-Triazol-3-yl)phenyl-sulfonamid |
| 1.10 | $Q_{10}$ | 2-(1,2-Oxazol-5-yl)phenyl-sulfonamid |
| 1.11 | $Q_{11}$ | 2-(1,2-Oxazol-3-yl)phenyl-sulfonamid |
| 1.12 | $Q_{12}$ | 2-(1,2-Oxazol-4-yl)phenyl-sulfonamid |
| 1.13 | $Q_{13}$ | 2-(1,3-Oxazol-2-yl)phenyl-sulfonamid |
| 1.14 | $Q_{14}$ | 2-(1,3-Oxazol-5-yl)phenyl-sulfonamid |
| 1.15 | $Q_{15}$ | 2-(1,3-Oxazol-4-yl)phenyl-sulfonamid |
| 1.16 | $Q_{16}$ | 2-(1,2-Thiazol-5-yl)phenyl-sulfonamid |
| 1.17 | $Q_{17}$ | 2-(1,2-Thiazol-3-yl)phenyl-sulfonamid |
| 1.18 | $Q_{18}$ | 2-(1,2-Thiazol-4-yl)phenyl-sulfonamid |
| 1.19 | $Q_{19}$ | 2-(1,3-Thiazol-2-yl)phenyl-sulfonamid |
| 1.20 | $Q_{20}$ | 2-(1,3-Thiazol-5-yl)phenyl-sulfonamid |
| 1.21 | $Q_{21}$ | 2-(1,3-Thiazol-4-yl)phenyl-sulfonamid |

Tabelle 1: (Fortsetzung)

| Verbindung Nr. | Ringsystem Nr. | |
|---|---|---|
| 1.22 | $Q_{22}$ | 2-(2-Methyl-pyrazol-5-yl)phenyl-sulfonamid |
| 1.23 | $Q_{23}$ | 2-(2-Methyl-pyrazol-4-yl)phenyl-sulfonamid |
| 1.24 | $Q_{24}$ | 2-(Pyrazol-5-yl)phenyl-sulfonamid |
| 1.25 | $Q_{25}$ | 2-(1-Methyl-imidazol-5-yl)phenyl-sulfonamid |
| 1.26 | $Q_{26}$ | 2-(1-Methyl-imidazol-2-yl)phenyl-sulfonamid |
| 1.27 | $Q_{27}$ | 2-(Imidazol-2-yl)phenyl-sulfonamid |
| 1.28 | $Q_{28}$ | 2-(Pyridazin-3-yl)phenyl-sulfonamid |
| 1.29 | $Q_{29}$ | 2-(Pyridazin-4-yl)phenyl-sulfonamid |
| 1.30 | $Q_{30}$ | 2-(Pyrimidin-2-yl)phenyl-sulfonamid |
| 1.31 | $Q_{31}$ | 2-(Pyrimidin-5-yl)phenyl-sulfonamid |
| 1.32 | $Q_{32}$ | 2-(Pyrazin-2-yl)phenyl-sulfonamid |
| 1.33 | $Q_{33}$ | 2-(1,3,5-Triazin-2-yl)phenyl-sulfonamid |
| 1.34 | $Q_{34}$ | 2-(1,2,4-Triazin-5-yl)phenyl-sulfonamid |
| 1.35 | $Q_{35}$ | 2-(1,2,4-Triazin-6-yl)phenyl-sulfonamid |
| 1.36 | $Q_{36}$ | 2-(1,2,4-Triazin-3-yl)phenyl-sulfonamid |
| 1.37 | $Q_{37}$ | 2-(Thiophen-2-yl)phenyl-sulfonamid |
| 1.38 | $Q_{38}$ | 2-(Furan-2-yl)phenyl-sulfonamid |
| 1.39 | $Q_{39}$ | 2-(1-Methylpyrrol-2-yl)phenyl-sulfonamid |
| 1.40 | $Q_{40}$ | 2-(Pyrrol-2-yl)phenyl-sulfonamid |
| 1.41 | $Q_{41}$ | 2-(1,3-Oxazolidin-2-yl)phenyl-sulfonamid |
| 1.42 | $Q_{42}$ | 2-(1,3-Thiazolidin-2-yl)phenyl-sulfonamid |
| 1.43 | $Q_{43}$ | 2-(Pyridin-2-yl)phenyl-sulfonamid |
| 1.44 | $Q_{44}$ | 2-(Pyridin-3-yl)phenyl-sulfonamid |
| 1.45 | $Q_{45}$ | 2-(Pyridin-4-yl)phenyl-sulfonamid |
| 1.46 | $Q_{46}$ | 2-(1-Methyl-imidazolin-2-yl)phenyl-sulfonamid |
| 1.47 | $Q_{47}$ | 2-(Imidazolin-2-yl)phenyl-sulfonamid |
| 1.48 | $Q_{48}$ | 2-(1,3-Thiazolin-2-yl)phenyl-sulfonamid |
| 1.49 | $Q_{49}$ | 2-(5-Methyl-1,3,4-oxadiazol-2-yl)phenyl-sulfonamid |
| 1.50 | $Q_{50}$ | 2-(1,3,4-Oxadiazol-2-yl)phenyl-sulfonamid |

Tabelle 1: (Fortsetzung)

| Verbindung Nr. | Ringsystem Nr. | |
|---|---|---|
| 1.51 | $Q_{51}$ | 2-(1,2,3-Thiadiazol-4-yl)-phenylsulfonamid |
| 1.52 | $Q_{52}$ | 2-(1,3-Oxazolin-2-yl)-phenylsulfonamid |
| 1.53 | $Q_{53}$ | 2-(Thiophen-2-yl)-6-chlorphenyl-sulfonamid |
| 1.54 | $Q_{54}$ | 2-(Furan-2-yl)-6-chlorphenyl-sulfonamid |
| 1.55 | $Q_{55}$ | 2-(5-Isopropyl-thiophen-2-yl)phenyl-sulfonamid |
| 1.56 | $Q_{56}$ | 2-(5-Isopropyl-furan-2-yl)phenyl-sulfonamid |
| 1.57 | $Q_{57}$ | 2-(5-n-Propyl-thiophen-2-yl)phenyl-sulfonamid |
| 1.58 | $Q_{58}$ | 2-(5-n-Propyl-furan-2-yl)phenyl-sulfonamid |
| 1.59 | $Q_{59}$ | 2-(5-t.Butyl-thiophen-2-yl)phenyl-sulfonamid |
| 1.60 | $Q_{60}$ | 2-(5-t.Butyl-furan-2-yl)phenyl-sulfonamid |
| 1.61 | $Q_{61}$ | 2-(5-Methoxymethyl-thiophen-2-yl)phenylsulfonamid |
| 1.62 | $Q_{62}$ | 2-(5-Methoxymethyl-furan-2-yl)phenylsulfonamid |
| 1.63 | $Q_{63}$ | 2-(5-Trifluormethyl-thiophen-2-yl)phenylsulfonamid |
| 1.64 | $Q_{64}$ | 2-(5-Trifluormethyl-furan-2-yl)-phenylsulfonamid |
| 1.65 | $Q_{65}$ | 2-(5-Methyl-thiophen-2-yl)phenyl-sulfonamid |
| 1.66 | $Q_{66}$ | 2-(5-Methyl-furan-2-yl)phenyl-sulfonamid |
| 1.67 | $Q_{67}$ | 2-(5-Ethyl-thiophen-2-yl)phenyl-sulfonamid |
| 1.68 | $Q_{68}$ | 2-(5-Ethyl-furan-2-yl)phenyl-sulfonamid |
| 1.69 | $Q_{69}$ | 2-(5-Trifluormethyl-1,3,4-Oxadiazol-2-yl) phenyl-sulfonamid |
| 1.70 | $Q_{70}$ | 2-(5-n-Propyl-1,3,4-oxadiazol-2-yl)phenylsulfonamid |
| 1.71 | $Q_{71}$ | 2-(5-i-Propyl-1,3,4-oxadiazol-2-yl)phenylsulfonamid |

Tabelle 1 : (Fortsetzung)

| Verbindung Nr. | Ringsystem Nr. | |
|---|---|---|
| 1.72 | $Q_{72}$ | 2-(5-t-Butyl-1,3,4-oxadiazol-2-yl)phenyl-sulfonamid |
| 1.73 | $Q_{73}$ | 2-(5-sec.Butyl-1,3,4-oxadiazol-2-yl)-phenyl-sulfonamid |
| 1.74 | $Q_{74}$ | 2-(5-iso-Butyl-1,3,4-oxadiazol-2-yl)-phenyl-sulfonamid |
| 1.75 | $Q_{75}$ | 2-(5-n-Butyl-1,3,4-oxadiazol-2-yl)phenyl-sulfonamid |
| 1.76 | $Q_{76}$ | 2-(5-Chlormethyl-1,3,4-oxadiazol-2-yl)-phenyl-sulfonamid |
| 1.77 | $Q_{77}$ | 2-(5-Methoxymethyl-1,3,4-oxadiazol-2-yl)phenyl-sulfonamid |
| 1.78 | $Q_{78}$ | 2-(5-Dimethylamino-1,3,4-oxadiazol-2-yl)-phenyl-sulfonamid |
| 1.79 | $Q_{79}$ | 2-(5-Cyano-1,3,4-oxadiazol-2-yl)phenyl-sulfonamid |
| 1.80 | $Q_{80}$ | 2-(5-Nitro-1,3,4-oxadiazol-2-yl)phenyl-sulfonamid |
| 1.81 | $Q_{81}$ | 2-(1-Methyl-4-chlor-pyrazol-5-yl)phenyl-sulfonamid |
| 1.82 | $Q_{82}$ | 2-(5-Methyl-1,2,4-oxadiazol-3-yl)phenyl-sulfonamid |
| 1.83 | $Q_{83}$ | 2-(5-t-Butyl-1,2,4-oxadiazol-3-yl)phenyl-sulfonamid |
| 1.84 | $Q_{84}$ | 2-(5-Ethyl-1,3,4-oxadiazol-2-yl)phenyl-sulfonamid |

Tabelle 2:

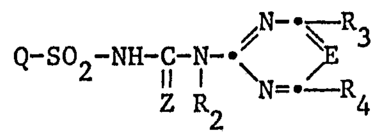

Q hat die in der Tabelle 1 gegebenen Bedeutungen für den anellierten Phenylring.

| Verb. Nr. | Q | $R_2$ | $R_3$ | $R_4$ | Z | E | Smp. [°C] |
|---|---|---|---|---|---|---|---|
| 2.1 | $Q_1$ | H | $CH_3$ | $OCHF_2$ | O | CH | |
| 2.2 | $Q_1$ | H | $OCHF_2$ | $OCHF_2$ | O | CH | |
| 2.3 | $Q_1$ | H | $OCHF_2$ | $-N(CH_3)_2$ | O | CH | |
| 2.4 | $Q_1$ | H | $OCH_3$ | $OCHF_2$ | O | CH | |
| 2.5 | $Q_1$ | H | $OCHF_2$ | $C_2H_5$ | O | CH | |
| 2.6 | $Q_1$ | H | $OCHF_2$ | Cl | O | CH | |
| 2.7 | $Q_1$ | H | $OCHF_2$ | $OCHF_2$ | O | CH | |
| 2.8 | $Q_1$ | H | $OCHF_2$ | $CF_3$ | O | CH | |
| 2.9 | $Q_2$ | H | $OCHF_2$ | $OCHF_2$ | O | CH | |
| 2.10 | $Q_2$ | H | $OCHF_2$ | $OCH_2CH_2F$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.11 | $Q_2$ | H | $OCH_3$ | cyclopropyl | O | N | |
| 2.12 | $Q_2$ | H | $OCH_3$ | cyclopropyl | O | CH | |
| 2.13 | $Q_2$ | H | cyclopropyl | $-OCH_2-CH_3$ | O | N | |
| 2.14 | $Q_2$ | H | $OC_2H_5$ | $OC_2H_5$ | O | CH | |
| 2.15 | $Q_2$ | H | $OCHF_2$ | $-OCH_2-CF_3$ | O | N | |
| 2.16 | $Q_2$ | H | $C_2H_5$ | $OC_2H_5$ | O | CH | |
| 2.17 | $Q_2$ | H | $-OCH_2-CH_3$ | $-OCH_2-CH_3$ | O | N | |
| 2.18 | $Q_2$ | H | $SCH_3$ | $OCHF_2$ | O | N | |
| 2.19 | $Q_2$ | H | $CH_3$ | $OCHF_2$ | S | CH | |
| 2.20 | $Q_2$ | $CH_3$ | $CH_3$ | $OCHF_2$ | O | CH | |
| 2.21 | $Q_2$ | H | $CH_3$ | $OCHF_2$ | O | CH | |
| 2.22 | $Q_2$ | H | $OCHF_2$ | $-N(CH_3)_2$ | O | N | |
| 2.23 | $Q_2$ | H | $OC_2H_5$ | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.24 | $Q_2$ | H | $OCH_3$ | $OCHF_2$ | O | CH | |
| 2.25 | $Q_2$ | H | $OCHF_2$ | Cl | O | CH | |
| 2.26 | $Q_2$ | H | $OCHF_2$ | F | O | CH | |
| 2.27 | $Q_2$ | H | $OCHF_2$ | $-NHCH_3$ | O | CH | |
| 2.28 | $Q_2$ | H | $CH_2F$ | $OCHF_2$ | O | CH | |
| 2.29 | $Q_2$ | H | $OCHF_2$ | $-N(CH_3)_2$ | O | CH | |
| 2.30 | $Q_2$ | H | $OCH_3$ | $-OCF_2-CHF_2$ | O | CH | |
| 2.31 | $Q_2$ | H | $CH_3$ | $-OCF_2-CHF_2$ | O | CH | |
| 2.32 | $Q_2$ | $CH_3$ | $OCH_3$ | $OCHF_2$ | O | CH | |
| 2.33 | $Q_2$ | H | $C_2H_5$ | $OC_2H_5$ | O | N | |
| 2.34 | $Q_3$ | H | $CH_3$ | $OCH_3$ | O | N | 190-191° |
| 2.35 | $Q_3$ | H | $OCH_3$ | $OCH_3$ | O | N | 190° |
| 2.36 | $Q_3$ | H | $OCH_3$ | $-N(CH_3)_2$ | O | CH | 203 - 205° |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.37 | $Q_3$ | H | $OCH_3$ | $-OCH_2-CF_3$ | O | N | 165° |
| 2.38 | $Q_3$ | H | $CH_3$ | $CH_3$ | O | CH | 201-203° |
| 2.39 | $Q_3$ | H | $OCH_3$ | $CH_3$ | O | CH | 195-196° Bsp. 1 |
| 2.40 | $Q_3$ | H | $OCH_3$ | $OCH_3$ | O | CH | 225° |
| 2.41 | $Q_3$ | H | $CH_3$ | $OCHF_2$ | O | CH | 149° |
| 2.42 | $Q_3$ | H | Cl | $OCH_3$ | O | CH | 190-192° |
| 2.43 | $Q_3$ | H | Cl | $CH_3$ | O | CH | 208° |
| 2.44 | $Q_3$ | H | $OCH_3$ | $CH_2OCH_3$ | O | CH | 201° |
| 2.45 | $Q_3$ | H | $OCH_3$ | $-N(CH_3)_2$ | O | N | 206° |
| 2.46 | $Q_4$ | H | cyclopropyl | $OCH_3$ | O | CH | |
| 2.47 | $Q_4$ | H | $OCHF_2$ | $OCHF_2$ | O | CH | |
| 2.48 | $Q_4$ | H | $OCH_3$ | $OCHF_2$ | O | CH | |
| 2.49 | $Q_4$ | H | $CH_3$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.50 | $Q_5$ | H | $C_2H_5$ | $OC_2H_5$ | O | N | |
| 2.51 | $Q_5$ | H | $OCHF_2$ | $OCHF_2$ | O | CH | |
| 2.52 | $Q_5$ | H | $OCHF_2$ | $-N(CH_3)_2$ | O | CH | |
| 2.53 | $Q_5$ | H | $OCHF_2$ | $-OCH_2-CF_3$ | O | N | |
| 2.54 | $Q_5$ | H | $OCH_3$ | cyclopropyl | O | N | |
| 2.55 | $Q_5$ | H | $OCH_3$ | cyclopropyl | O | CH | |
| 2.56 | $Q_5$ | H | $OCH_3$ | $OCHF_2$ | O | CH | |
| 2.57 | $Q_5$ | H | $CH_3$ | $OCHF_2$ | O | CH | |
| 2.58 | $Q_6$ | H | $C_2H_5$ | $OC_2H_5$ | O | N | |
| 2.59 | $Q_6$ | H | $OCHF_2$ | $OCHF_2$ | O | CH | |
| 2.60 | $Q_6$ | H | $OCHF_2$ | $-N(CH_3)_2$ | O | CH | |
| 2.61 | $Q_6$ | H | $OCHF_2$ | $-OCH_2-CF_3$ | O | N | |
| 2.62 | $Q_6$ | H | $OCH_3$ | cyclopropyl | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.63 | $Q_6$ | H | $OCH_3$ | cyclopropyl | O | CH | |
| 2.64 | $Q_6$ | H | $OCH_3$ | $OCHF_2$ | O | CH | |
| 2.65 | $Q_6$ | H | $CH_3$ | $OCHF_2$ | O | CH | |
| 2.66 | $Q_7$ | H | $CH_3$ | $OCH_3$ | O | N | 203-205° |
| 2.67 | $Q_7$ | H | $OCHF_2$ | $OCHF_2$ | O | CH | |
| 2.68 | $Q_7$ | H | $OCHF_2$ | $-N(CH_3)_2$ | O | CH | |
| 2.69 | $Q_7$ | H | $OCHF_2$ | $-OCH_2-CF_3$ | O | N | |
| 2.70 | $Q_7$ | H | $CH_3$ | $CH_3$ | O | CH | 219° |
| 2.71 | $Q_7$ | H | $OCH_3$ | $CH_3$ | O | CH | 226-227° |
| 2.72 | $Q_7$ | H | $OCH_3$ | $OCH_3$ | O | CH | 205° |
| 2.73 | $Q_7$ | H | $CH_3$ | $OCHF_2$ | O | CH | |
| 2.74 | $Q_8$ | H | $CH_3$ | $OCH_3$ | O | N | |
| 2.75 | $Q_8$ | H | $OCHF_2$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.76 | $Q_8$ | H | $OCHF_2$ | $-N(CH_3)_2$ | O | N | |
| 2.77 | $Q_8$ | H | $OCH_3$ | cyclopropyl | O | N | |
| 2.78 | $Q_8$ | H | $OCH_3$ | cyclopropyl | O | CH | |
| 2.79 | $Q_8$ | H | $OCH_3$ | $OCHF_2$ | O | CH | |
| 2.80 | $Q_8$ | H | $OC_2H_5$ | $OC_2H_5$ | O | CH | |
| 2.81 | $Q_8$ | H | $CH_3$ | $OCHF_2$ | O | CH | |
| 2.82 | $Q_9$ | H | $C_2H_5$ | $OC_2H_5$ | O | N | |
| 2.83 | $Q_9$ | H | $OCHF_2$ | $OCHF_2$ | O | CH | |
| 2.84 | $Q_9$ | H | $OCHF_2$ | $-N(CH_3)_2$ | O | CH | |
| 2.85 | $Q_9$ | H | $OCH_3$ | cyclopropyl | O | CH | |
| 2.86 | $Q_9$ | H | $OCH_3$ | cyclopropyl | O | CH | |
| 2.87 | $Q_9$ | H | $OCH_3$ | $OCHF_2$ | O | CH | |
| 2.88 | $Q_9$ | H | $OC_2H_5$ | $OC_2H_5$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.89 | $Q_9$ | H | $CH_3$ | $OCHF_2$ | O | CH | |
| 2.90 | $Q_{10}$ | H | $CH_3$ | $OCH_3$ | O | N | 185-187° |
| 2.91 | $Q_{10}$ | H | $OCHF_2$ | $OCHF_2$ | O | CH | |
| 2.92 | $Q_{10}$ | H | $OCHF_2$ | $-N(CH_3)_2$ | O | CH | |
| 2.93 | $Q_{10}$ | H | $OCH_3$ | cyclopropyl | O | N | |
| 2.94 | $Q_{10}$ | H | $CH_3$ | $CH_3$ | O | CH | 207-209° |
| 2.95 | $Q_{10}$ | H | $OCH_3$ | $CH_3$ | O | CH | 215-217° |
| 2.96 | $Q_{10}$ | H | $OCH_3$ | $OCH_3$ | O | CH | 197-199° |
| 2.97 | $Q_{10}$ | H | $CH_3$ | $OCHF_2$ | O | CH | 176-178° |
| 2.98 | $Q_{11}$ | H | $C_2H_5$ | $OC_2H_5$ | O | N | |
| 2.99 | $Q_{11}$ | H | $OCHF_2$ | $OCHF_2$ | O | CH | |
| 2.100 | $Q_{11}$ | H | $OCHF_2$ | $-N(CH_3)_2$ | O | CH | |
| 2.101 | $Q_{11}$ | H | $OCH_3$ | cyclopropyl | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.102 | $Q_{11}$ | H | $OCH_3$ | cyclopropyl | O | CH | |
| 2.103 | $Q_{11}$ | H | $OCH_3$ | $OCHF_2$ | O | CH | |
| 2.104 | $Q_{11}$ | H | $OC_2H_5$ | $OC_2H_5$ | O | CH | |
| 2.105 | $Q_{11}$ | H | $CH_3$ | $OCHF_2$ | O | CH | |
| 2.106 | $Q_{12}$ | H | $C_2H_5$ | $OC_2H_5$ | O | N | |
| 2.107 | $Q_{12}$ | H | $OCHF_2$ | $OCHF_2$ | O | CH | |
| 2.108 | $Q_{12}$ | H | $OCHF_2$ | $-N(CH_3)_2$ | O | N | |
| 2.109 | $Q_{12}$ | H | $OCH_3$ | cyclopropyl | O | CH | |
| 2.110 | $Q_{12}$ | H | $OCH_3$ | cyclopropyl | O | CH | |
| 2.111 | $Q_{12}$ | H | $OCH_3$ | $OCHF_2$ | O | CH | |
| 2.112 | $Q_{12}$ | H | $OC_2H_5$ | $OC_2H_5$ | O | CH | |
| 2.113 | $Q_{12}$ | H | $CH_3$ | $OCHF_2$ | O | CH | |
| 2.114 | $Q_{13}$ | H | $C_2H_5$ | $OC_2H_5$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.115 | $Q_{13}$ | H | $OCHF_2$ | $OCHF_2$ | O | CH | |
| 2.116 | $Q_{13}$ | H | $OCHF_2$ | $-N(CH_3)_2$ | O | CH | |
| 2.117 | $Q_{13}$ | H | $OCH_3$ | cyclopropyl | O | N | |
| 2.118 | $Q_{13}$ | H | $OCH_3$ | cyclopropyl | O | CH | |
| 2.119 | $Q_{13}$ | H | $OCH_3$ | $OCHF_2$ | O | CH | |
| 2.120 | $Q_{13}$ | H | $OC_2H_5$ | $OC_2H_5$ | O | CH | |
| 2.121 | $Q_{13}$ | H | $CH_3$ | $OCHF_2$ | O | CH | |
| 2.122 | $Q_{14}$ | H | $CH_3$ | $OCH_3$ | O | N | 180-181° |
| 2.123 | $Q_{14}$ | H | $OCH_3$ | Cl | O | CH | 199-201° |
| 2.124 | $Q_{14}$ | H | $OCH_3$ | cyclopropyl | O | N | |
| 2.125 | $Q_{14}$ | H | $OCH_3$ | cyclopropyl | O | CH | |
| 2.126 | $Q_{14}$ | H | $CH_3$ | $CH_3$ | O | CH | 220-221° |
| 2.127 | $Q_{14}$ | H | $OCH_3$ | $CH_3$ | O | CH | 210-211° |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.128 | $Q_{14}$ | H | $OCH_3$ | $OCH_3$ | O | CH | 207-209° |
| 2.129 | $Q_{14}$ | H | $CH_3$ | $OCHF_2$ | O | CH | 182° |
| 2.130 | $Q_{15}$ | H | $C_2H_5$ | $OC_2H_5$ | O | N | |
| 2.131 | $Q_{15}$ | H | $OCHF_2$ | $OCHF_2$ | O | CH | |
| 2.132 | $Q_{15}$ | H | $OCH_3$ | cyclopropyl | O | N | |
| 2.133 | $Q_{15}$ | H | $OCH_3$ | cyclopropyl | O | CH | |
| 2.134 | $Q_{15}$ | H | $OCH_3$ | $OCHF_2$ | O | CH | |
| 2.135 | $Q_{15}$ | H | $OC_2H_5$ | $OC_2H_5$ | O | N | |
| 2.136 | $Q_{15}$ | H | $OC_2H_5$ | $OC_2H_5$ | O | CH | |
| 2.137 | $Q_{15}$ | H | $CH_3$ | $OCHF_2$ | O | CH | |
| 2.138 | $Q_{16}$ | H | $C_2H_5$ | $OC_2H_5$ | O | N | |
| 2.139 | $Q_{16}$ | H | $OCHF_2$ | $OCHF_2$ | O | N | |
| 2.140 | $Q_{16}$ | H | $OCH_3$ | cyclopropyl | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.141 | $Q_{16}$ | H | $OCH_3$ | cyclopropyl | O | CH | |
| 2.142 | $Q_{16}$ | H | $OCH_3$ | $OCHF_2$ | O | CH | |
| 2.143 | $Q_{16}$ | H | $OC_2H_5$ | $OC_2H_5$ | O | CH | |
| 2.144 | $Q_{16}$ | H | $OCH_3$ | $OCH_3$ | O | CH | |
| 2.145 | $Q_{16}$ | H | $CH_3$ | $OCHF_2$ | O | CH | |
| 2.146 | $Q_{17}$ | H | $C_2H_5$ | $OC_2H_5$ | O | N | |
| 2.147 | $Q_{17}$ | H | $OCHF_2$ | $OCHF_2$ | O | CH | |
| 2.148 | $Q_{17}$ | H | $OCH_3$ | cyclopropyl | O | N | |
| 2.149 | $Q_{17}$ | H | $OCH_3$ | cyclopropyl | O | CH | |
| 2.150 | $Q_{17}$ | H | $OCH_3$ | $OCHF_2$ | O | CH | |
| 2.151 | $Q_{17}$ | H | $C_2H_5$ | $OC_2H_5$ | O | N | |
| 2.152 | $Q_{17}$ | H | $OC_2H_5$ | $OC_2H_5$ | O | CH | |
| 2.153 | $Q_{17}$ | H | $CH_3$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.154 | $Q_{18}$ | H | $C_2H_5$ | $OC_2H_5$ | O | N | |
| 2.155 | $Q_{18}$ | H | $OCHF_2$ | $OCHF_2$ | O | CH | |
| 2.156 | $Q_{18}$ | H | $OCH_3$ | cyclopropyl | O | N | |
| 2.157 | $Q_{18}$ | H | $OCH_3$ | cyclopropyl | O | CH | |
| 2.158 | $Q_{18}$ | H | $OCH_3$ | $OCHF_2$ | O | CH | |
| 2.159 | $Q_{18}$ | H | $OC_2H_5$ | $C_2H_5$ | O | CH | |
| 2.160 | $Q_{18}$ | H | $OC_2H_5$ | $OC_2H_5$ | O | CH | |
| 2.161 | $Q_{18}$ | H | $CH_3$ | $OCHF_2$ | O | CH | |
| 2.162 | $Q_{19}$ | H | $C_2H_5$ | $OC_2H_5$ | O | N | |
| 2.163 | $Q_{19}$ | H | $OCHF_2$ | $OCHF_2$ | O | CH | |
| 2.164 | $Q_{19}$ | H | $OCH_3$ | cyclopropyl | O | N | |
| 2.165 | $Q_{19}$ | H | $OCH_3$ | cyclopropyl | O | CH | |
| 2.166 | $Q_{19}$ | H | $CH_3$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.167 | $Q_{19}$ | H | $OC_2H_5$ | $C_2H_5$ | O | CH | |
| 2.168 | $Q_{19}$ | H | $OC_2H_5$ | $OC_2H_5$ | O | CH | |
| 2.169 | $Q_{19}$ | H | $CH_3$ | $-OCHF_2$ | O | CH | |
| 2.170 | $Q_{20}$ | H | $C_2H_5$ | $OC_2H_5$ | O | N | |
| 2.171 | $Q_{20}$ | H | $OCHF_2$ | $OCHF_2$ | O | CH | |
| 2.172 | $Q_{20}$ | H | $OCH_3$ | cyclopropyl | O | N | |
| 2.173 | $Q_{20}$ | H | $OCH_3$ | cyclopropyl | O | CH | |
| 2.174 | $Q_{20}$ | H | $OCH_3$ | $OCHF_2$ | O | CH | |
| 2.175 | $Q_{20}$ | H | $OC_2H_5$ | $C_2H_5$ | O | CH | |
| 2.176 | $Q_{20}$ | H | $OC_2H_5$ | $OC_2H_5$ | O | CH | |
| 2.177 | $Q_{20}$ | H | $CH_3$ | $OCHF_2$ | O | CH | |
| 2.178 | $Q_{21}$ | H | $C_2H_5$ | $OC_2H_5$ | O | N | |
| 2.179 | $Q_{21}$ | H | $OCHF_2$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.180 | $Q_{21}$ | H | $OCH_3$ | cyclopropyl | O | CH | |
| 2.181 | $Q_{21}$ | H | $OCH_3$ | cyclopropyl | O | N | |
| 2.182 | $Q_{21}$ | H | $OCH_3$ | $C_2H_5$ | O | CH | |
| 2.183 | $Q_{21}$ | H | $OCH_3$ | $CH_3$ | O | CH | |
| 2.184 | $Q_{21}$ | H | $OC_2H_5$ | $OC_2H_5$ | O | CH | |
| 2.185 | $Q_{21}$ | H | $CH_3$ | $OCHF_2$ | O | CH | |
| 2.186 | $Q_{22}$ | H | $C_2H_5$ | $OC_2H_5$ | O | N | |
| 2.187 | $Q_{22}$ | H | $OCHF_2$ | $OCHF_2$ | O | N | |
| 2.188 | $Q_{22}$ | H | $OCH_3$ | cyclopropyl | O | N | |
| 2.189 | $Q_{22}$ | H | $OCH_3$ | cyclopropyl | O | CH | |
| 2.190 | $Q_{22}$ | H | $OCH_3$ | $OCHF_2$ | O | CH | |
| 2.191 | $Q_{22}$ | H | $OC_2H_5$ | $C_2H_5$ | O | CH | |
| 2.192 | $Q_{22}$ | H | $OC_2H_5$ | $OC_2H_5$ | O | CH | |
| 2.193 | $Q_{22}$ | H | $CH_3$ | $OCHF_2$ | O | CH | |

<u>Tabelle 2</u> (Fortsetzung)

| Verb. Nr. | Q | $R_2$ | $R_3$ | $R_4$ | Z | E | Smp. [°C] |
|---|---|---|---|---|---|---|---|
| 2.194 | $Q_{23}$ | H | $C_2H_5$ | $OC_2H_5$ | O | N | |
| 2.195 | $Q_{23}$ | H | $OCHF_2$ | $OCHF_2$ | O | N | |
| 2.196 | $Q_{23}$ | H | $OCH_3$ | cyclopropyl | O | N | |
| 2.197 | $Q_{23}$ | H | $OCH_3$ | cyclopropyl | O | CH | |
| 2.198 | $Q_{23}$ | H | $OCH_3$ | $OCHF_2$ | O | CH | |
| 2.199 | $Q_{23}$ | H | $OC_2H_5$ | $C_2H_5$ | O | CH | |
| 2.200 | $Q_{23}$ | H | $OC_2H_5$ | $OC_2H_5$ | O | CH | |
| 2.201 | $Q_{23}$ | H | $CH_3$ | $OCHF_2$ | O | CH | |
| 2.202 | $Q_{24}$ | H | $C_2H_5$ | $OC_2H_5$ | O | N | |
| 2.203 | $Q_{24}$ | H | $OCHF_2$ | $OCHF_2$ | O | CH | |
| 2.204 | $Q_{24}$ | H | $OCH_3$ | cyclopropyl | O | N | |
| 2.205 | $Q_{24}$ | H | $OCH_3$ | cyclopropyl | O | CH | |
| 2.206 | $Q_{24}$ | H | $OCH_3$ | $OCHF_2$ | O | CH | |
| 2.207 | $Q_{24}$ | H | $OC_2H_5$ | $C_2H_5$ | O | CH | |
| 2.208 | $Q_{24}$ | H | $OC_2H_5$ | $OC_2H_5$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.209 | $Q_{24}$ | H | $CH_3$ | $OCHF_2$ | O | CH | |
| 2.210 | $Q_{25}$ | H | $C_2H_5$ | $OC_2H_5$ | O | N | |
| 2.211 | $Q_{25}$ | H | $OCHF_2$ | $OCHF_2$ | O | CH | |
| 2.212 | $Q_{25}$ | H | $OCH_3$ | cyclopropyl | O | N | |
| 2.213 | $Q_{25}$ | H | $OCH_3$ | cyclopropyl | O | CH | |
| 2.214 | $Q_{25}$ | H | $OCH_3$ | $OCHF_2$ | O | CH | |
| 2.215 | $Q_{25}$ | H | $OC_2H_5$ | $C_2H_5$ | O | CH | |
| 2.216 | $Q_{25}$ | H | $OC_2H_5$ | $OC_2H_5$ | O | CH | |
| 2.217 | $Q_{25}$ | H | $CH_3$ | $OCHF_2$ | O | CH | |
| 2.218 | $Q_{26}$ | H | $C_2H_5$ | $OC_2H_5$ | O | N | |
| 2.219 | $Q_{26}$ | H | $OCHF_2$ | $OCHF_2$ | O | CH | |
| 2.220 | $Q_{26}$ | H | $OCH_3$ | cyclopropyl | O | N | |
| 2.221 | $Q_{26}$ | H | $OCH_3$ | cyclopropyl | O | CH | |
| 2.222 | $Q_{26}$ | H | $OCH_3$ | $OCHF_2$ | O | CH | |
| 2.223 | $Q_{26}$ | H | $OC_2H_5$ | $C_2H_5$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.224 | $Q_{26}$ | H | $OC_2H_5$ | $OC_2H_5$ | O | CH | |
| 2.225 | $Q_{26}$ | H | $CH_3$ | $OCHF_2$ | O | CH | |
| 2.226 | $Q_{27}$ | H | $C_2H_5$ | $OC_2H_5$ | O | N | |
| 2.227 | $Q_{27}$ | H | $OCHF_2$ | $OCHF_2$ | O | CH | |
| 2.228 | $Q_{27}$ | H | $OCH_3$ | cyclopropyl | O | N | |
| 2.229 | $Q_{27}$ | H | $OCH_3$ | cyclopropyl | O | CH | |
| 2.230 | $Q_{27}$ | H | $OCH_3$ | $OCHF_2$ | O | CH | |
| 2.231 | $Q_{27}$ | H | $OC_2H_5$ | $C_2H_5$ | O | CH | |
| 2.232 | $Q_{27}$ | H | $OC_2H_5$ | $OC_2H_5$ | O | CH | |
| 2.233 | $Q_{27}$ | H | $CH_3$ | $OCHF_2$ | O | CH | |
| 2.234 | $Q_{28}$ | H | $C_2H_5$ | $OC_2H_5$ | O | N | |
| 2.235 | $Q_{28}$ | H | $OCHF_2$ | $OCHF_2$ | O | CH | |
| 2.236 | $Q_{28}$ | H | $OCH_3$ | cyclopropyl | O | N | |
| 2.237 | $Q_{28}$ | H | $OCH_3$ | cyclopropyl | O | CH | |
| 2.238 | $Q_{28}$ | H | $OCH_3$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.239 | $Q_{28}$ | H | $OC_2H_5$ | $C_2H_5$ | O | CH | |
| 2.240 | $Q_{28}$ | H | $OC_2H_5$ | $OC_2H_5$ | O | CH | |
| 2.241 | $Q_{28}$ | H | $CH_3$ | $OCHF_2$ | O | CH | |
| 2.242 | $Q_{29}$ | H | $C_2H_5$ | $OC_2H_5$ | O | N | |
| 2.243 | $Q_{29}$ | H | $OCHF_2$ | $OCHF_2$ | O | CH | |
| 2.244 | $Q_{29}$ | H | $OCH_3$ | cyclopropyl | O | N | |
| 2.245 | $Q_{29}$ | H | $OCH_3$ | cyclopropyl | O | CH | |
| 2.246 | $Q_{29}$ | H | $OCH_3$ | $OCHF_2$ | O | CH | |
| 2.247 | $Q_{29}$ | H | $OC_2H_5$ | $C_2H_5$ | O | CH | |
| 2.248 | $Q_{29}$ | H | $OC_2H_5$ | $OC_2H_5$ | O | CH | |
| 2.249 | $Q_{29}$ | H | $CH_3$ | $OCHF_2$ | O | CH | |
| 2.250 | $Q_{49}$ | H | $CH_3$ | $CH_3$ | O | CH | 195° |
| 2.251 | $Q_{50}$ | H | $CH_3$ | $CH_3$ | O | CH | 210° |
| 2.252 | $Q_{50}$ | H | $CH_3$ | $OCH_3$ | O | N | 174-175° |
| 2.253 | $Q_{50}$ | H | $CH_3$ | $OCH_3$ | O | CH | 203-205° |
| 2.254 | $Q_{50}$ | H | $OCH_3$ | $OCH_3$ | O | CH | 201-202° |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.255 | $Q_{37}$ | H | $OCHF_2$ | $CH_3$ | O | CH | 177-179° |
| 2.256 | $Q_{37}$ | $CH_3$ | $OCHF_2$ | $CH_3$ | O | CH | |
| 2.257 | $Q_{37}$ | H | $OCHF_2$ | $OCHF_2$ | O | CH | |
| 2.258 | $Q_{37}$ | H | $OCHF_2$ | $OCHF_2$ | S | CH | |
| 2.259 | $Q_{37}$ | H | $OCHF_2$ | $CH_3$ | S | CH | |
| 2.260 | $Q_{37}$ | H | $OCHF_2$ | $N(CH_3)_2$ | O | CH | |
| 2.261 | $Q_{37}$ | H | $OCHF_2$ | $N(CH_3)C_2H_5$ | O | CH | |
| 2.262 | $Q_{37}$ | H | $OCHF_2$ | $C_2H_5$ | O | CH | |
| 2.263 | $Q_{37}$ | H | $OCHF_2$ | $OCH_3$ | O | CH | |
| 2.264 | $Q_{37}$ | H | $OCHF_2$ | Cl | O | CH | |
| 2.265 | $Q_{37}$ | H | $OCHF_2$ | $CH_2F$ | O | CH | |
| 2.266 | $Q_{37}$ | H | $OCHF_2$ | $CF_3$ | O | CH | |
| 2.267 | $Q_{37}$ | H | $OCHF_2$ | F | O | CH | |
| 2.268 | $Q_{37}$ | H | $OCHF_2$ | Br | O | CH | |
| 2.269 | $Q_{37}$ | H | $OCHF_2$ | $OCH_2CH_2F$ | O | CH | |
| 2.270 | $Q_{37}$ | H | $OCHF_2$ | $OCH_2CF_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.271 | $Q_{37}$ | H | $OCH_3$ | $CH_3$ | O | CH | 231-232° Zers. |
| 2.272 | $Q_{37}$ | H | $OC_2H_5$ | $O_2H_5$ | O | CH | |
| 2.273 | $Q_{37}$ | H | $OC_2H_5$ | $OC_2H_5$ | O | CH | |
| 2.274 | $Q_{37}$ | H | $OCH_3$ | $CH_3$ | O | N | 211-212° Zers. |
| 2.275 | $Q_{37}$ | H | $OC_2H_5$ | $OC_2H_5$ | O | N | |
| 2.276 | $Q_{37}$ | H | cyclopropyl | $OCH_3$ | O | CH | |
| 2.277 | $Q_{37}$ | H | cyclopropyl | $OCH_3$ | O | N | |
| 2.278 | $Q_{37}$ | H | cyclopropyl | $OC_2H_5$ | O | CH | |
| 2.279 | $Q_{37}$ | H | $OCH_3$ | $OCH_3$ | O | CH | 238-239° |
| 2.280 | $Q_{38}$ | H | $OCHF_2$ | $CH_3$ | O | CH | |
| 2.281 | $Q_{38}$ | $CH_3$ | $OCHF_2$ | $CH_3$ | O | CH | |
| 2.282 | $Q_{38}$ | H | $OCHF_2$ | $OCHF_2$ | O | CH | |
| 2.283 | $Q_{38}$ | H | $OCHF_2$ | $OCHF_2$ | S | CH | |
| 2.284 | $Q_{38}$ | $CH_3$ | $OCHF_2$ | $OCHF_2$ | O | CH | |
| 2.285 | $Q_{38}$ | H | $OCHF_2$ | $CH_3$ | S | CH | |
| 2.286 | $Q_{38}$ | H | $OCHF_2$ | $N(CH_3)_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.287 | $Q_{38}$ | H | $OCHF_2$ | $C_2H_5$ | O | CH | |
| 2.288 | $Q_{38}$ | H | $OCHF_2$ | $OCH_3$ | O | CH | |
| 2.289 | $Q_{38}$ | H | $OCHF_2$ | $CH_2F$ | O | CH | |
| 2.290 | $Q_{38}$ | H | $OCHF_2$ | $CF_3$ | O | CH | |
| 2.291 | $Q_{38}$ | H | $OCHF_2$ | $OCH_2CH_2F$ | O | CH | |
| 2.292 | $Q_{38}$ | H | $OCHF_2$ | $OCH_2CF_3$ | O | CH | |
| 2.293 | $Q_{38}$ | H | $OC_2H_5$ | $C_2H_5$ | O | CH | |
| 2.294 | $Q_{38}$ | H | $OC_2H_5$ | $OC_2H_5$ | O | CH | |
| 2.295 | $Q_{38}$ | H | $OC_2H_5$ | $C_2H_5$ | O | N | |
| 2.296 | $Q_{38}$ | H | $OC_2H_5$ | $OC_2H_5$ | O | N | |
| 2.297 | $Q_{38}$ | H | cyclopropyl | $OCH_3$ | O | CH | |
| 2.298 | $Q_{38}$ | H | cyclopropyl | $OCH_3$ | O | N | |
| 2.299 | $Q_{38}$ | H | cyclopropyl | $OC_2H_5$ | O | CH | |
| 2.300 | $Q_{38}$ | H | cyclopropyl | $OC_2H_5$ | O | N | |
| 2.301 | $Q_{38}$ | H | $CH_2OC_2H_5$ | $OCH_3$ | O | CH | |
| 2.302 | $Q_{53}$ | H | $OCHF_2$ | $CH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.303 | $Q_{53}$ | H | $OCHF_2$ | $CH_3$ | S | CH | |
| 2.304 | $Q_{53}$ | $CH_3$ | $OCHF_2$ | $CH_3$ | O | CH | |
| 2.305 | $Q_{53}$ | H | $OCHF_2$ | $OCHF_2$ | O | CH | |
| 2.306 | $Q_{53}$ | $CH_3$ | $OCHF_2$ | $OCHF_2$ | O | CH | |
| 2.307 | $Q_{53}$ | H | $OCHF_2$ | $N(CH_3)_2$ | O | CH | |
| 2.308 | $Q_{53}$ | H | $OCHF_2$ | $C_2H_5$ | O | CH | |
| 2.309 | $Q_{53}$ | H | $OCHF_2$ | $OCH_3$ | O | CH | |
| 2.310 | $Q_{53}$ | H | $OCHF_2$ | $CH_2F$ | O | CH | |
| 2.311 | $Q_{53}$ | H | $OC_2H_5$ | $OC_2H_5$ | O | CH | |
| 2.312 | $Q_{53}$ | H | $OC_2H_5$ | $C_2H_5$ | O | CH | |
| 2.313 | $Q_{53}$ | H | $OC_2H_5$ | $C_2H_5$ | O | N | |
| 2.314 | $Q_{53}$ | H | $OC_2H_5$ | $OC_2H_5$ | O | N | |
| 2.315 | $Q_{53}$ | H | cyclopropyl | $OCH_3$ | O | CH | |
| 2.316 | $Q_{53}$ | H | cyclopropyl | $OCH_3$ | O | N | |
| 2.317 | $Q_{54}$ | H | $OCHF_2$ | $CH_3$ | O | CH | |
| 2.318 | $Q_{54}$ | H | $OCHF_2$ | $CHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.319 | $Q_{54}$ | H | $OCHF_2$ | $N(CH_3)_2$ | O | CH | |
| 2.320 | $Q_{54}$ | H | $OCHF_2$ | $OCH_3$ | O | CH | |
| 2.321 | $Q_{54}$ | H | $OCHF_2$ | $CH_2F$ | O | CH | |
| 2.322 | $Q_{54}$ | H | $OC_2H_5$ | $OC_2H_5$ | O | CH | |
| 2.323 | $Q_{54}$ | H | $OC_2H_5$ | $OC_2H_5$ | O | N | |
| 2.324 | $Q_{54}$ | H | $OC_2H_5$ | $C_2H_5$ | O | CH | |
| 2.325 | $Q_{54}$ | H | cyclopropyl | $OCH_3$ | O | CH | |
| 2.326 | $Q_{54}$ | H | cyclopropyl | $OCH_3$ | O | N | |
| 2.327 | $Q_{55}$ | H | $CH_3$ | $OCH_3$ | O | CH | |
| 2.328 | $Q_{55}$ | H | $CH_3$ | $OCHF_2$ | O | CH | |
| 2.329 | $Q_{55}$ | H | $OCH_3$ | $OCH_3$ | O | CH | |
| 2.330 | $Q_{55}$ | H | $CH_3$ | $CH_3$ | O | CH | |
| 2.331 | $Q_{55}$ | H | $CH_3$ | $OCH_3$ | O | N | |
| 2.332 | $Q_{55}$ | H | $OCH_3$ | $OCH_3$ | O | N | |
| 2.333 | $Q_{56}$ | H | $CH_3$ | $OCH_3$ | O | CH | |
| 2.334 | $Q_{56}$ | H | $CH_3$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.335 | $Q_{56}$ | H | $OCH_3$ | $OCH_3$ | O | CH | |
| 2.336 | $Q_{56}$ | H | $CH_3$ | $CH_3$ | O | CH | |
| 2.337 | $Q_{56}$ | H | $CH_3$ | $OCH_3$ | O | N | |
| 2.338 | $Q_{56}$ | H | $OCH_3$ | $OCH_3$ | O | N | |
| 2.339 | $Q_{57}$ | H | $CH_3$ | $OCH_3$ | O | CH | |
| 2.340 | $Q_{57}$ | H | $CH_3$ | $OCHF_2$ | O | CH | |
| 2.341 | $Q_{57}$ | H | $OCH_3$ | $OCH_3$ | O | CH | |
| 2.342 | $Q_{57}$ | H | $CH_3$ | $CH_3$ | O | CH | |
| 2.343 | $Q_{57}$ | H | $CH_3$ | $OCH_3$ | O | N | |
| 2.344 | $Q_{57}$ | H | $OCH_3$ | $OCH_3$ | O | N | |
| 2.345 | $Q_{58}$ | H | $CH_3$ | $CH_3$ | O | CH | |
| 2.346 | $Q_{58}$ | H | $OCH_3$ | $OCH_3$ | O | CH | |
| 2.347 | $Q_{58}$ | H | $OCH_3$ | $OCH_3$ | O | CH | |
| 2.348 | $Q_{58}$ | H | $CH_3$ | $OCH_3$ | O | N | |
| 2.349 | $Q_{58}$ | H | $OCH_3$ | $OCH_3$ | O | N | |
| 2.350 | $Q_{59}$ | H | $CH_3$ | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.351 | $Q_{59}$ | H | $CH_3$ | $CH_3$ | O | CH | |
| 2.352 | $Q_{59}$ | H | $CH_3$ | $OCHF_2$ | O | CH | |
| 2.353 | $Q_{59}$ | H | $OCH_3$ | $OCH_3$ | O | CH | |
| 2.354 | $Q_{59}$ | H | $CH_3$ | $OCH_3$ | O | N | |
| 2.355 | $Q_{59}$ | H | $OCH_3$ | $OCH_3$ | O | N | |
| 2.356 | $Q_{60}$ | H | $CH_3$ | $CH_3$ | O | CH | |
| 2.357 | $Q_{60}$ | H | $CH_3$ | $OCH_3$ | O | CH | |
| 2.358 | $Q_{60}$ | H | $OCH_3$ | $OCH_3$ | O | CH | |
| 2.359 | $Q_{60}$ | H | $CH_3$ | $OCH_3$ | O | N | |
| 2.360 | $Q_{60}$ | H | $OCH_3$ | $OCH_3$ | O | N | |
| 2.361 | $Q_{61}$ | H | $CH_3$ | $OCH_3$ | O | CH | |
| 2.362 | $Q_{61}$ | H | $CH_3$ | $OCH_3$ | O | CH | |
| 2.363 | $Q_{61}$ | H | $OCH_3$ | $OCH_3$ | O | CH | |
| 2.364 | $Q_{61}$ | H | $CH_3$ | $CH_3$ | O | CH | |
| 2.365 | $Q_{61}$ | H | $CH_3$ | $OCH_3$ | O | N | |
| 2.366 | $Q_{62}$ | H | $CH_3$ | $CH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.367 | $Q_{62}$ | H | $CH_3$ | $OCH_3$ | O | CH | |
| 2.368 | $Q_{62}$ | H | $OCH_3$ | $OCH_3$ | O | CH | |
| 2.369 | $Q_{62}$ | H | $CH_3$ | $OCH_3$ | O | N | |
| 2.370 | $Q_{62}$ | H | $OCH_3$ | $OCH_3$ | O | N | |
| 2.371 | $Q_{63}$ | H | $CH_3$ | $CH_3$ | O | CH | |
| 2.372 | $Q_{63}$ | H | $CH_3$ | $OCH_3$ | O | CH | |
| 2.373 | $Q_{63}$ | H | $OCH_3$ | $OCH_3$ | O | CH | |
| 2.374 | $Q_{63}$ | H | $CH_3$ | $OCH_3$ | O | N | |
| 2.375 | $Q_{63}$ | H | $OCH_3$ | $OCH_3$ | O | N | |
| 2.376 | $Q_{64}$ | H | $CH_3$ | $OCH_3$ | O | CH | |
| 2.377 | $Q_{64}$ | H | $CH_3$ | $OCH_3$ | O | CH | |
| 2.378 | $Q_{64}$ | H | $OCH_3$ | $OCH_3$ | O | CH | |
| 2.379 | $Q_{64}$ | H | $OCH_3$ | $OCH_3$ | O | N | |
| 2.380 | $Q_{64}$ | H | $CH_3$ | $OCH_3$ | O | N | |
| 2.381 | $Q_{65}$ | H | $OCHF_2$ | $CH_3$ | O | CH | |
| 2.382 | $Q_{65}$ | H | $OCHF_2$ | $CH_3$ | S | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.383 | $Q_{65}$ | H | $OCHF_2$ | $OCHF_2$ | O | CH | |
| 2.384 | $Q_{65}$ | $CH_3$ | $OCHF_2$ | $OCHF_2$ | O | CH | |
| 2.385 | $Q_{65}$ | $CH_3$ | $OCHF_2$ | $OCHF_2$ | S | CH | |
| 2.386 | $Q_{65}$ | H | $OCHF_2$ | $N(CH_3)_2$ | O | CH | |
| 2.387 | $Q_{65}$ | H | $OCHF_2$ | $OCH_3$ | O | CH | |
| 2.388 | $Q_{65}$ | H | $OCHF_2$ | $CH_2F$ | O | CH | |
| 2.389 | $Q_{65}$ | H | $OC_2H_5$ | $OC_2H_5$ | O | CH | |
| 2.390 | $Q_{65}$ | H | $OC_2H_5$ | $C_2H_5$ | O | CH | |
| 2.391 | $Q_{65}$ | H | $OC_2H_5$ | $OC_2H_5$ | O | N | |
| 2.392 | $Q_{65}$ | H | $OC_2H_5$ | $C_2H_5$ | O | N | |
| 2.393 | $Q_{65}$ | H | cyclopropyl | $OCH_3$ | O | CH | |
| 2.394 | $Q_{65}$ | H | cyclopropyl | $OCH_3$ | O | N | |
| 2.395 | $Q_{66}$ | H | $OCHF_2$ | $CH_3$ | O | CH | |
| 2.396 | $Q_{66}$ | H | $OCHF_2$ | $OCHF_2$ | O | CH | |
| 2.397 | $Q_{66}$ | H | $OCHF_2$ | $N(CH_3)_2$ | O | CH | |
| 2.398 | $Q_{66}$ | H | $OCHF_2$ | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.399 | $Q_{66}$ | H | $OCHF_2$ | $CH_2F$ | O | CH | |
| 2.400 | $Q_{66}$ | H | $OC_2H_5$ | $OC_2H_5$ | O | CH | |
| 2.401 | $Q_{66}$ | H | $OC_2H_5$ | $C_2H_5$ | O | CH | |
| 2.402 | $Q_{66}$ | H | $OC_2H_5$ | $OC_2H_5$ | O | N | |
| 2.403 | $Q_{66}$ | H | $OC_2H_5$ | $C_2H_5$ | O | N | |
| 2.404 | $Q_{66}$ | H | cyclopropyl | $OCH_3$ | O | CH | |
| 2.405 | $Q_{66}$ | H | cyclopropyl | $OCH_3$ | O | N | |
| 2.406 | $Q_{66}$ | H | cyclopropyl | $OC_2H_5$ | O | $CH_3$ | |
| 2.407 | $Q_{67}$ | H | $OCHF_2$ | $CH_3$ | O | CH | |
| 2.408 | $Q_{67}$ | H | $OCHF_2$ | $CH_3$ | S | CH | |
| 2.409 | $Q_{67}$ | H | $OCHF_2$ | $OCH_3$ | O | CH | |
| 2.410 | $Q_{67}$ | $CH_3$ | $OCHF_2$ | $OCHF_2$ | O | CH | |
| 2.411 | $Q_{67}$ | $CH_3$ | $OCHF_2$ | $OCHF_2$ | S | CH | |
| 2.412 | $Q_{67}$ | H | $OCHF_2$ | $N(CH_3)_2$ | O | CH | |
| 2.413 | $Q_{67}$ | H | $OCHF_2$ | $OCH_3$ | O | CH | |
| 2.414 | $Q_{67}$ | H | $OCHF_2$ | $CH_2F$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.415 | $Q_{67}$ | H | $OC_2H_5$ | $OC_2H_5$ | O | CH | |
| 2.416 | $Q_{67}$ | H | $OC_2H_5$ | $C_2H_5$ | O | CH | |
| 2.417 | $Q_{67}$ | H | $OC_2H_5$ | $OC_2H_5$ | O | N | |
| 2.418 | $Q_{67}$ | H | $OC_2H_5$ | $C_2H_5$ | O | N | |
| 2.419 | $Q_{67}$ | H | cyclopropyl | $OCH_3$ | O | CH | |
| 2.420 | $Q_{67}$ | H | cyclopropyl | $OCH_3$ | O | N | |
| 2.421 | $Q_{68}$ | H | $OCHF_2$ | $CH_3$ | O | CH | |
| 2.422 | $Q_{68}$ | H | $OCHF_2$ | $OCHF_2$ | O | CH | |
| 2.423 | $Q_{68}$ | H | $OCHF_2$ | $N(CH_3)_2$ | O | CH | |
| 2.424 | $Q_{68}$ | H | $OCHF_2$ | $OCH_3$ | O | CH | |
| 2.425 | $Q_{68}$ | H | $OCHF_2$ | $CHF_2$ | O | CH | |
| 2.426 | $Q_{68}$ | H | $OC_2H_5$ | $OC_2H_5$ | O | CH | |
| 2.427 | $Q_{68}$ | H | $OC_2H_5$ | $C_2H_5$ | O | CH | |
| 2.428 | $Q_{68}$ | H | $OC_2H_5$ | $OC_2H_5$ | O | N | |
| 2.429 | $Q_{68}$ | H | $OC_2H_5$ | $C_2H_5$ | O | N | |
| 2.430 | $Q_{68}$ | H | cyclopropyl | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.431 | $Q_{68}$ | H | cyclopropyl | $OCH_3$ | O | N | |
| 2.432 | $Q_{68}$ | H | cyclopropyl | $OC_2H_5$ | O | CH | |
| 2.433 | $Q_{69}$ | H | $CH_3$ | $CH_3$ | O | CH | 180° |
| 2.434 | $Q_{69}$ | H | $OCH_3$ | $OCH_3$ | O | CH | 170-173° |
| 2.435 | $Q_{69}$ | H | $CH_3$ | $OCH_3$ | O | CH | 183-184° |
| 2.436 | $Q_{69}$ | H | $CH_3$ | $OCHF_2$ | O | CH | |
| 2.437 | $Q_{69}$ | H | $CH_3$ | $OCH_3$ | O | N | 172-174° |
| 2.438 | $Q_{69}$ | H | $OCH_3$ | $OCH_3$ | O | N | |
| 2.439 | $Q_{69}$ | H | $CH_3$ | $CH_3$ | O | N | |
| 2.440 | $Q_{69}$ | H | $OCHF_2$ | $OCHF_2$ | O | CH | |
| 2.441 | $Q_{69}$ | H | $CH_3$ | Cl | O | CH | |
| 2.442 | $Q_{69}$ | H | $OCH_3$ | Cl | O | CH | |
| 2.443 | $Q_{69}$ | H | $OC_2H_5$ | $C_2H_5$ | O | CH | |
| 2.444 | $Q_{69}$ | H | $OC_2H_5$ | $CH_3$ | O | CH | |
| 2.445 | $Q_{69}$ | H | $OC_2H_5$ | $CH_3$ | O | N | |
| 2.446 | $Q_{69}$ | H | $OC_2H_5$ | $C_2H_5$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.447 | $Q_{69}$ | H | cyclopropyl | $OCH_3$ | O | CH | |
| 2.448 | $Q_{69}$ | H | cyclopropyl | $OCH_3$ | O | N | |
| 2.449 | $Q_{69}$ | $CH_3$ | $CH_3$ | $OCH_3$ | O | CH | |
| 2.450 | $Q_{69}$ | $CH_3$ | $CH_3$ | $OCH_3$ | O | N | |
| 2.451 | $Q_{69}$ | H | $CH_3$ | $OCH_3$ | S | CH | |
| 2.452 | $Q_{69}$ | H | $CH_3$ | $OCH_3$ | S | N | |
| 2.453 | $Q_{69}$ | $CH_3$ | $CH_3$ | $OCH_3$ | S | N | |
| 2.454 | $Q_{69}$ | $CH_3$ | $OCH_3$ | $OCH_3$ | O | N | |
| 2.455 | $Q_{69}$ | H | $OCH_3$ | $N(CH_3)_2$ | O | N | |
| 2.456 | $Q_{69}$ | H | $CH_3$ | $C_2H_5$ | O | N | |
| 2.457 | $Q_{69}$ | H | $OC_2H_5$ | $O_2H_5$ | O | N | |
| 2.458 | $Q_{69}$ | H | $OC_2H_5$ | $OCH_3$ | O | N | |
| 2.459 | $Q_{69}$ | H | $OCH_3$ | $OCH(CH_3)_2$ | O | N | |
| 2.460 | $Q_{69}$ | H | $OCH_3$ | $SCH_3$ | O | N | |
| 2.461 | $Q_{69}$ | H | $OCH_3$ | $OC_2H_4OCH_3$ | O | N | |
| 2.462 | $Q_{69}$ | H | $CH_3$ | $CH_2Cl$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.463 | $Q_{69}$ | H | $OCH_3$ | $C_2H_5$ | O | N | |
| 2.464 | $Q_{69}$ | H | $OCH_3$ | $OCH_2CF_3$ | O | N | |
| 2.465 | $Q_{69}$ | H | $OCH_3$ | $CF_3$ | O | N | |
| 2.466 | $Q_{69}$ | H | $OCH_3$ | $CH_2OCH_3$ | O | CH | |
| 2.467 | $Q_{69}$ | H | $OCH_3$ | $OCHF_2$ | O | CH | |
| 2.468 | $Q_{69}$ | H | Cl | $OCHF_2$ | O | CH | |
| 2.469 | $Q_{69}$ | H | $OCHF_2$ | $N(CH_3)_2$ | O | CH | |
| 2.470 | $Q_{69}$ | H | $OC_2H_5$ | $OCHF_2$ | O | CH | |
| 2.471 | $Q_{69}$ | H | $OCHF_2$ | $CH_2F$ | O | CH | |
| 2.472 | $Q_{69}$ | H | $OCH_3$ | $N(CH_3)_2$ | O | CH | |
| 2.473 | $Q_{69}$ | H | $OCH_3$ | $SCH_3$ | O | CH | |
| 2.474 | $Q_{69}$ | H | $OCH_3$ | $SCHF_2$ | O | CH | |
| 2.475 | $Q_{69}$ | H | $OCH_3$ | $CH_2CF_3$ | O | CH | |
| 2.476 | $Q_{69}$ | H | $OCH_3$ | $CH_2Cl$ | O | CH | |
| 2.477 | $Q_{70}$ | H | $CH_3$ | $CH_3$ | O | CH | |
| 2.478 | $Q_{70}$ | H | $OCH_3$ | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.479 | $Q_{70}$ | H | $CH_3$ | $OCH_3$ | O | CH | |
| 2.480 | $Q_{70}$ | H | $CH_3$ | $OCHF_2$ | O | CH | |
| 2.481 | $Q_{70}$ | H | $CH_3$ | $OCH_3$ | O | N | |
| 2.482 | $Q_{70}$ | H | $CH_3$ | $OCH_3$ | O | N | |
| 2.483 | $Q_{70}$ | H | $OCH_3$ | $CH_3$ | O | N | |
| 2.484 | $Q_{70}$ | H | $OCHF_2$ | $OCHF_2$ | O | CH | |
| 2.485 | $Q_{70}$ | H | $CH_3$ | Cl | O | CH | |
| 2.486 | $Q_{70}$ | H | $OCH_3$ | Cl | O | CH | |
| 2.487 | $Q_{70}$ | H | $OC_2H_5$ | $C_2H_5$ | O | CH | |
| 2.488 | $Q_{70}$ | H | $OC_2H_5$ | $CH_3$ | O | CH | |
| 2.489 | $Q_{70}$ | H | $OC_2H_5$ | $CH_3$ | O | N | |
| 2.490 | $Q_{70}$ | H | $OC_2H_5$ | $C_2H_5$ | O | N | |
| 2.491 | $Q_{70}$ | H | cyclopropyl | $OCH_3$ | O | CH | |
| 2.492 | $Q_{70}$ | H | cyclopropyl | $OCH_3$ | O | N | |
| 2.493 | $Q_{70}$ | $CH_3$ | $CH_3$ | $OCH_3$ | O | CH | |
| 2.494 | $Q_{70}$ | $CH_3$ | $CH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.495 | $Q_{70}$ | H | $CH_3$ | $OCH_3$ | S | CH | |
| 2.496 | $Q_{70}$ | H | $CH_3$ | $OCH_3$ | S | N | |
| 2.497 | $Q_{70}$ | $CH_3$ | $CH_3$ | $OCH_3$ | S | N | |
| 2.498 | $Q_{70}$ | $CH_3$ | $OCH_3$ | $OCH_3$ | O | N | |
| 2.499 | $Q_{70}$ | H | $OCH_3$ | $N(CH_3)_2$ | O | N | |
| 2.500 | $Q_{70}$ | H | $OCH_3$ | $C_2H_5$ | O | N | |
| 2.501 | $Q_{70}$ | H | $OC_2H_5$ | $OC_2H_5$ | O | N | |
| 2.502 | $Q_{70}$ | H | $OC_2H_5$ | $OCH_3$ | O | N | |
| 2.503 | $Q_{70}$ | H | $OC_2H_5$ | $OCH(CH_3)_2$ | O | N | |
| 2.504 | $Q_{70}$ | H | $OCH_3$ | $SCH_3$ | O | N | |
| 2.505 | $Q_{70}$ | H | $OCH_3$ | $OC_2H_4OCH_3$ | O | N | |
| 2.506 | $Q_{70}$ | H | $CH_3$ | $CH_2Cl$ | O | N | |
| 2.507 | $Q_{70}$ | H | $OCH_3$ | $C_2H_5$ | O | N | |
| 2.508 | $Q_{70}$ | H | $OCH_3$ | $OCH_2CF_3$ | O | N | |
| 2.509 | $Q_{70}$ | H | $OCH_3$ | $CF_3$ | O | N | |
| 2.510 | $Q_{70}$ | H | $OCH_3$ | $CH_2OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.511 | $Q_{70}$ | H | $OCH_3$ | $OCHF_2$ | O | CH | |
| 2.512 | $Q_{70}$ | H | Cl | $OCHF_2$ | O | CH | |
| 2.513 | $Q_{70}$ | H | $OCHF_2$ | $N(CH_3)_2$ | O | CH | |
| 2.514 | $Q_{70}$ | H | $OC_2H_5$ | $OCHF_2$ | O | CH | |
| 2.515 | $Q_{70}$ | H | $OCHF_2$ | $OCH_2F$ | O | CH | |
| 2.516 | $Q_{70}$ | H | $OCH_3$ | $N(CH_3)_2$ | O | CH | |
| 2.517 | $Q_{70}$ | H | $OCH_3$ | $SCH_3$ | O | CH | |
| 2.518 | $Q_{70}$ | H | $OCH_3$ | $SCHF_2$ | O | CH | |
| 2.519 | $Q_{70}$ | H | $OCH_3$ | $CH_2CF_3$ | O | CH | |
| 2.520 | $Q_{70}$ | H | $OCH_3$ | $CH_2Cl$ | O | CH | |
| 2.521 | $Q_{71}$ | H | $CH_3$ | $CH_3$ | O | CH | |
| 2.522 | $Q_{71}$ | H | $OCH_3$ | $OCH_3$ | O | CH | |
| 2.523 | $Q_{71}$ | H | $CH_3$ | $OCH_3$ | O | CH | |
| 2.524 | $Q_{71}$ | H | $CH_3$ | $OCHF_2$ | O | CH | |
| 2.525 | $Q_{71}$ | H | $CH_3$ | $OCH_3$ | O | N | |
| 2.526 | $Q_{71}$ | H | $OCH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.527 | $Q_{71}$ | H | $CH_3$ | $CH_3$ | O | N | |
| 2.528 | $Q_{71}$ | H | $OCHF_2$ | $OCHF_2$ | O | CH | |
| 2.529 | $Q_{71}$ | H | $CH_3$ | Cl | O | CH | |
| 2.530 | $Q_{71}$ | H | $OCH_3$ | Cl | O | CH | |
| 2.531 | $Q_{71}$ | H | $OC_2H_5$ | $C_2H_5$ | O | CH | |
| 2.532 | $Q_{71}$ | H | $OC_2H_5$ | $CH_3$ | O | CH | |
| 2.533 | $Q_{71}$ | H | $OC_2H_5$ | $CH_3$ | O | CH | |
| 2.534 | $Q_{71}$ | H | $OC_2H_5$ | $CH_3$ | O | N | |
| 2.535 | $Q_{71}$ | H | cyclopropyl | $OCH_3$ | O | CH | |
| 2.536 | $Q_{71}$ | H | cyclopropyl | $OCH_3$ | O | N | |
| 2.537 | $Q_{71}$ | $CH_3$ | $CH_3$ | $OCH_3$ | O | CH | |
| 2.538 | $Q_{71}$ | $CH_3$ | $CH_3$ | $OCH_3$ | O | N | |
| 2.539 | $Q_{71}$ | H | $CH_3$ | $OCH_3$ | S | CH | |
| 2.540 | $Q_{71}$ | H | $CH_3$ | $OCH_3$ | S | N | |
| 2.541 | $Q_{71}$ | $CH_3$ | $CH_3$ | $OCH_3$ | S | N | |
| 2.542 | $Q_{71}$ | $CH_3$ | $OCH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.543 | $Q_{71}$ | H | $OCH_3$ | $C_2H_5$ | O | N | |
| 2.544 | $Q_{71}$ | H | $OC_2H_5$ | $OC_2H_5$ | O | N | |
| 2.545 | $Q_{71}$ | H | $OCH_3$ | $OC_2H_5$ | O | N | |
| 2.546 | $Q_{71}$ | H | $OCH_3$ | $OC_2H_5$ | O | N | |
| 2.547 | $Q_{71}$ | H | $OCH_3$ | $OCH(CH_3)_2$ | O | N | |
| 2.548 | $Q_{71}$ | H | $OCH_3$ | $SCH_3$ | O | N | |
| 2.549 | $Q_{71}$ | H | $OCH_3$ | $OC_2H_4OCH_3$ | O | N | |
| 2.550 | $Q_{71}$ | H | $CH_3$ | $CH_2Cl$ | O | N | |
| 2.551 | $Q_{71}$ | H | $OCH_3$ | $C_2H_5$ | O | N | |
| 2.552 | $Q_{71}$ | H | $OCH_3$ | $CH_2CF_3$ | O | N | |
| 2.553 | $Q_{71}$ | H | $OCH_3$ | $CF_3$ | O | N | |
| 2.554 | $Q_{71}$ | H | $OCH_3$ | $CH_2OCH_3$ | O | CH | |
| 2.555 | $Q_{71}$ | H | $OCH_3$ | $OCHF_2$ | O | CH | |
| 2.556 | $Q_{71}$ | H | Cl | $OCHF_2$ | O | CH | |
| 2.557 | $Q_{71}$ | H | $OCHF_2$ | $N(CH_3)_2$ | O | CH | |
| 2.558 | $Q_{71}$ | H | $OC_2H_5$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.559 | $Q_{71}$ | H | $OCHF_2$ | $CH_2F$ | O | CH | |
| 2.560 | $Q_{71}$ | H | $OCH_3$ | $N(CH_3)_2$ | O | CH | |
| 2.561 | $Q_{71}$ | H | $OCH_3$ | $SCH_3$ | O | CH | |
| 2.562 | $Q_{71}$ | H | $OCH_3$ | $SCHF_2$ | O | CH | |
| 2.563 | $Q_{71}$ | H | $OCH_3$ | $CH_2CF_3$ | O | CH | |
| 2.564 | $Q_{71}$ | H | $OCH_3$ | $CH_2Cl$ | O | CH | |
| 2.565 | $Q_{72}$ | H | $CH_3$ | $CH_3$ | O | CH | 182-184° |
| 2.566 | $Q_{72}$ | H | $OCH_3$ | $OCH_3$ | O | CH | 210-212° |
| 2.567 | $Q_{72}$ | H | $CH_3$ | $OCH_3$ | O | CH | 185° |
| 2.568 | $Q_{72}$ | H | $CH_3$ | $OCHF_2$ | O | CH | 178-179° |
| 2.569 | $Q_{72}$ | H | $CH_3$ | $OCH_3$ | O | N | 196-198° |
| 2.570 | $Q_{72}$ | H | $OCH_3$ | $OCH_3$ | O | N | |
| 2.571 | $Q_{72}$ | H | $CH_3$ | $CH_3$ | O | N | |
| 2.572 | $Q_{72}$ | H | $OCHF_2$ | $OCHF_2$ | O | CH | |
| 2.573 | $Q_{72}$ | H | $CH_3$ | Cl | O | CH | |
| 2.574 | $Q_{72}$ | H | $OCH_3$ | Cl | O | CH | 191-193° |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.575 | $Q_{72}$ | H | $OC_2H_5$ | $C_2H_5$ | O | CH | |
| 2.576 | $Q_{72}$ | H | $OC_2H_5$ | $CH_3$ | O | CH | |
| 2.577 | $Q_{72}$ | H | $OC_2H_5$ | $CH_3$ | O | N | |
| 2.578 | $Q_{72}$ | H | $OC_2H_5$ | $C_2H_5$ | O | N | |
| 2.579 | $Q_{72}$ | H | cyclopropyl | $OCH_3$ | O | CH | |
| 2.580 | $Q_{72}$ | H | cyclopropyl | $OCH_3$ | O | N | |
| 2.581 | $Q_{72}$ | $CH_3$ | $CH_3$ | $OCH_3$ | O | CH | |
| 2.582 | $Q_{72}$ | $CH_3$ | $CH_3$ | $OCH_3$ | O | N | |
| 2.583 | $Q_{72}$ | H | $CH_3$ | $OCH_3$ | S | CH | |
| 2.584 | $Q_{72}$ | H | $CH_3$ | $OCH_3$ | S | N | |
| 2.585 | $Q_{72}$ | $CH_3$ | $CH_3$ | $OCH_3$ | S | N | |
| 2.586 | $Q_{72}$ | $CH_3$ | $OCH_3$ | $OCH_3$ | O | N | |
| 2.587 | $Q_{72}$ | H | $OCH_3$ | $N(CH_3)_2$ | O | N | |
| 2.588 | $Q_{72}$ | H | $CH_3$ | $C_2H_5$ | O | N | |
| 2.589 | $Q_{72}$ | H | $OC_2H_5$ | $OC_2H_5$ | O | N | |
| 2.590 | $Q_{72}$ | H | $OC_2H_5$ | $OC H_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.591 | $Q_{72}$ | H | $OCH_3$ | $OCH(CH_3)_2$ | O | N | |
| 2.592 | $Q_{72}$ | H | $OCH_3$ | $SCH_3$ | O | N | |
| 2.593 | $Q_{72}$ | H | $OCH_3$ | $OC_2H_4OCH_3$ | O | N | |
| 2.594 | $Q_{72}$ | H | $CH_3$ | $CH_2Cl$ | O | N | |
| 2.595 | $Q_{72}$ | H | $OCH_3$ | $C_2H_5$ | O | N | |
| 2.596 | $Q_{72}$ | H | $OCH_3$ | $OCH_2CF_3$ | O | N | |
| 2.597 | $Q_{72}$ | H | $OCH_3$ | $CF_3$ | O | N | |
| 2.598 | $Q_{72}$ | H | $OCH_3$ | $CH_2OCH_3$ | O | CH | |
| 2.599 | $Q_{72}$ | H | $OCH_3$ | $OCHF_2$ | O | CH | |
| 2.600 | $Q_{72}$ | H | Cl | $OCHF_2$ | O | CH | |
| 2.601 | $Q_{72}$ | H | $OCHF_2$ | $N(CH_3)_2$ | O | CH | |
| 2.602 | $Q_{72}$ | H | $OC_2H_5$ | $OCHF_2$ | O | CH | |
| 2.603 | $Q_{72}$ | H | $OCHF_2$ | $CH_2F$ | O | CH | |
| 2.604 | $Q_{72}$ | H | $OCH_3$ | $N(CH_3)_2$ | O | CH | |
| 2.605 | $Q_{72}$ | H | $OCH_3$ | $SCH_3$ | O | CH | |
| 2.606 | $Q_{72}$ | H | $OCH_3$ | $SCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.607 | $Q_{72}$ | H | $OCH_3$ | $CH_2CF_3$ | O | CH | |
| 2.608 | $Q_{72}$ | H | $OCH_3$ | $CH_2Cl$ | O | CH | |
| 2.609 | $Q_{73}$ | H | $CH_3$ | $CH_3$ | O | CH | |
| 2.610 | $Q_{73}$ | H | $OCH_3$ | $OCH_3$ | O | CH | |
| 2.611 | $Q_{73}$ | H | $CH_3$ | $OCH_3$ | O | CH | |
| 2.612 | $Q_{73}$ | H | $CH_3$ | $OCHF_2$ | O | CH | |
| 2.613 | $Q_{73}$ | H | $CH_3$ | $OCH_3$ | O | N | |
| 2.614 | $Q_{73}$ | H | $OCH_3$ | $OCH_3$ | O | N | |
| 2.615 | $Q_{73}$ | H | $CH_3$ | $CH_3$ | O | N | |
| 2.616 | $Q_{73}$ | H | $OCHF_2$ | $OCHF_2$ | O | CH | |
| 2.617 | $Q_{73}$ | H | $CH_3$ | Cl | O | CH | |
| 2.618 | $Q_{73}$ | H | $OCH_3$ | Cl | O | CH | |
| 2.619 | $Q_{73}$ | H | $OC_2H_5$ | $C_2H_5$ | O | CH | |
| 2.620 | $Q_{73}$ | H | $OC_2H_5$ | $CH_3$ | O | CH | |
| 2.621 | $Q_{73}$ | H | $OC_2H_5$ | $CH_3$ | O | N | |
| 2.622 | $Q_{73}$ | H | $OC_2H_5$ | $C_2H_5$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.623 | $Q_{73}$ | H | cyclopropyl | $OCH_3$ | O | CH | |
| 2.624 | $Q_{73}$ | H | cyclopropyl | $OCH_3$ | O | N | |
| 2.625 | $Q_{73}$ | $CH_3$ | $CH_3$ | $OCH_3$ | O | CH | |
| 2.626 | $Q_{73}$ | $CH_3$ | $CH_3$ | $CH_3$ | O | N | |
| 2.627 | $Q_{73}$ | H | $CH_3$ | $OCH_3$ | S | CH | |
| 2.628 | $Q_{73}$ | H | $CH_3$ | $OCH_3$ | S | N | |
| 2.629 | $Q_{73}$ | $CH_3$ | $CH_3$ | $OCH_3$ | S | N | |
| 2.630 | $Q_{73}$ | $CH_3$ | $OCH_3$ | $OCH_3$ | O | N | |
| 2.631 | $Q_{73}$ | H | $OCH_3$ | $N(CH_3)_2$ | O | N | |
| 2.632 | $Q_{73}$ | H | $CH_3$ | $C_2H_5$ | O | N | |
| 2.633 | $Q_{73}$ | H | $OC_2H_5$ | $OC_2H_5$ | O | N | |
| 2.634 | $Q_{73}$ | H | $OC_2H_5$ | $OCH_3$ | O | N | |
| 2.635 | $Q_{73}$ | H | $OCH_3$ | $OCH(CH_3)_2$ | O | N | |
| 2.636 | $Q_{73}$ | H | $OCH_3$ | $SCH_3$ | O | N | |
| 2.637 | $Q_{73}$ | H | $OCH_3$ | $OC_2H_4OCH_3$ | O | N | |
| 2.638 | $Q_{73}$ | H | $CH_3$ | $CH_2Cl$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.639 | $Q_{73}$ | H | $OCH_3$ | $C_2H_5$ | O | N | |
| 2.640 | $Q_{73}$ | H | $OCH_3$ | $OCH_2CF_3$ | O | N | |
| 2.641 | $Q_{73}$ | H | $OCH_3$ | $CF_3$ | O | N | |
| 2.642 | $Q_{73}$ | H | $OCH_3$ | $CH_2OCH_3$ | O | CH | |
| 2.643 | $Q_{73}$ | H | $OCH_3$ | $OCHF_2$ | O | CH | |
| 2.644 | $Q_{73}$ | H | Cl | $OCHF_2$ | O | CH | |
| 2.645 | $Q_{73}$ | H | $CHF_2$ | $N(CH_3)_2$ | O | CH | |
| 2.646 | $Q_{73}$ | H | $OC_2H_5$ | $OCHF_2$ | O | CH | |
| 2.647 | $Q_{73}$ | H | $OCHF_2$ | $CH_2F$ | O | CH | |
| 2.648 | $Q_{73}$ | H | $OCH_3$ | $N(CH_3)_2$ | O | CH | |
| 2.649 | $Q_{73}$ | H | $OCH_3$ | $SCH_3$ | O | CH | |
| 2.650 | $Q_{73}$ | H | $OCH_3$ | $SCHF_2$ | O | CH | |
| 2.651 | $Q_{73}$ | H | $OCH_3$ | $CH_2CF_3$ | O | CH | |
| 2.652 | $Q_{73}$ | H | $OCH_3$ | $CH_2Cl$ | O | CH | |
| 2.653 | $Q_{74}$ | H | $CH_3$ | $CH_3$ | O | CH | |
| 2.654 | $Q_{74}$ | H | $OCH_3$ | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.655 | $Q_{74}$ | H | $CH_3$ | $OCH_3$ | O | CH | |
| 2.656 | $Q_{74}$ | H | $CH_3$ | $OCHF_2$ | O | CH | |
| 2.657 | $Q_{74}$ | H | $CH_3$ | $OCH_3$ | O | N | |
| 2.658 | $Q_{74}$ | H | $OCH_3$ | $CH_3$ | O | N | |
| 2.659 | $Q_{74}$ | H | $CH_3$ | $CH_3$ | O | N | |
| 2.660 | $Q_{74}$ | H | $OCHF_2$ | $OCHF_2$ | O | CH | |
| 2.661 | $Q_{74}$ | H | $CH_3$ | Cl | O | CH | |
| 2.662 | $Q_{74}$ | H | $OCH_3$ | Cl | O | CH | |
| 2.663 | $Q_{74}$ | H | $OC_2H_5$ | $C_2H_5$ | O | CH | |
| 2.664 | $Q_{74}$ | H | $OC_2H_5$ | $CH_3$ | O | CH | |
| 2.665 | $Q_{74}$ | H | $OC_2H_5$ | $CH_3$ | O | N | |
| 2.666 | $Q_{74}$ | H | $OC_2H_5$ | $C_2H_5$ | O | N | |
| 2.667 | $Q_{74}$ | H | cyclopropyl | $OCH_3$ | O | CH | |
| 2.668 | $Q_{74}$ | H | cyclopropyl | $OCH_3$ | O | N | |
| 2.669 | $Q_{74}$ | $CH_3$ | $CH_3$ | $OCH_3$ | O | CH | |
| 2.670 | $Q_{74}$ | $CH_3$ | $CH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.671 | $Q_{74}$ | H | $CH_3$ | $OCH_3$ | S | CH | |
| 2.672 | $Q_{74}$ | H | $CH_3$ | $OCH_3$ | S | N | |
| 2.673 | $Q_{74}$ | $CH_3$ | $CH_3$ | $OCH_3$ | S | N | |
| 2.674 | $Q_{74}$ | $CH_3$ | $OCH_3$ | $OCH_3$ | O | N | |
| 2.675 | $Q_{74}$ | H | $OCH_3$ | $N(CH_3)_2$ | O | N | |
| 2.676 | $Q_{74}$ | H | $CH_3$ | $C_2H_5$ | O | N | |
| 2.677 | $Q_{74}$ | H | $OC_2H_5$ | $OC_2H_5$ | O | N | |
| 2.678 | $Q_{74}$ | H | $OC_2H_5$ | $OCH_3$ | O | N | |
| 2.679 | $Q_{74}$ | H | $OCH_3$ | $OCH(CH_3)_2$ | O | N | |
| 2.680 | $Q_{74}$ | H | $OCH_3$ | $SCH_3$ | O | N | |
| 2.681 | $Q_{74}$ | H | $OCH_3$ | $OC_2H_4OCH_3$ | O | N | |
| 2.682 | $Q_{74}$ | H | $CH_3$ | $CH_2Cl$ | O | N | |
| 2.683 | $Q_{74}$ | H | $OCH_3$ | $C_2H_5$ | O | N | |
| 2.684 | $Q_{74}$ | H | $OCH_3$ | $OCH_2CF_3$ | O | N | |
| 2.685 | $Q_{74}$ | H | $OCH_3$ | $CF_3$ | O | N | |
| 2.686 | $Q_{74}$ | H | $OCH_3$ | $CH_2OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.687 | $Q_{74}$ | H | Cl | $OCHF_2$ | O | CH | |
| 2.688 | $Q_{74}$ | H | Cl | $OCHF_2$ | O | CH | |
| 2.689 | $Q_{74}$ | H | $OCHF_2$ | $N(CH_3)_2$ | O | CH | |
| 2.690 | $Q_{74}$ | H | $OC_2H_5$ | $OCHF_2$ | O | CH | |
| 2.691 | $Q_{74}$ | H | $OCHF_2$ | $CH_2F$ | O | CH | |
| 2.692 | $Q_{74}$ | H | $OCH_3$ | $N(CH_3)_2$ | O | CH | |
| 2.693 | $Q_{74}$ | H | $OCH_3$ | $SCH_3$ | O | CH | |
| 2.694 | $Q_{74}$ | H | $OCH_3$ | $SCHF_2$ | O | CH | |
| 2.695 | $Q_{74}$ | H | $OCH_3$ | $CH_2CF_3$ | O | CH | |
| 2.696 | $Q_{74}$ | H | $OCH_3$ | $CH_2Cl$ | O | CH | |
| 2.697 | $Q_{75}$ | H | $CH_3$ | $CH_3$ | O | CH | |
| 2.698 | $Q_{75}$ | H | $OCH_3$ | $OCH_3$ | O | CH | |
| 2.699 | $Q_{75}$ | H | $CH_3$ | $OCH_3$ | O | CH | |
| 2.700 | $Q_{75}$ | H | $CH_3$ | $OCHF_2$ | O | CH | |
| 2.701 | $Q_{75}$ | H | $OCH_3$ | $CH_3$ | O | N | |
| 2.702 | $Q_{75}$ | H | $OCH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.703 | $Q_{75}$ | H | $CH_3$ | $CH_3$ | O | N | |
| 2.704 | $Q_{75}$ | H | $CH_3$ | Cl | O | CH | |
| 2.705 | $Q_{75}$ | H | $OCH_3$ | Cl | O | CH | |
| 2.706 | $Q_{75}$ | H | $OC_2H_5$ | $CH_3$ | O | CH | |
| 2.707 | $Q_{75}$ | H | $OC_2H_5$ | $CH_3$ | O | N | |
| 2.708 | $Q_{75}$ | H | cyclopropyl | $OCH_3$ | O | CH | |
| 2.709 | $Q_{75}$ | H | cyclopropyl | $OCH_3$ | O | N | |
| 2.710 | $Q_{75}$ | H | $OCH_3$ | $N(CH_3)_2$ | O | N | |
| 2.711 | $Q_{75}$ | H | $CH_3$ | $C_2H_5$ | O | N | |
| 2.712 | $Q_{75}$ | H | $OCH_3$ | $C_2H_5$ | O | N | |
| 2.713 | $Q_{75}$ | H | $OCH_3$ | $CH_2OCH_3$ | O | N | |
| 2.714 | $Q_{75}$ | H | $OCH_3$ | $OCHF_2$ | O | CH | |
| 2.715 | $Q_{75}$ | H | $OCH_3$ | $N(CH_3)_2$ | O | CH | |
| 2.716 | $Q_{75}$ | H | $OCH_3$ | $SCH_3$ | O | CH | |
| 2.717 | $Q_{75}$ | H | $OCH_3$ | $CH_2Cl$ | O | CH | |
| 2.718 | $Q_{76}$ | H | $CH_3$ | $CH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.719 | $Q_{76}$ | H | $CH_3$ | $OCH_3$ | O | CH | |
| 2.720 | $Q_{76}$ | H | $CH_3$ | $OCH_3$ | O | CH | |
| 2.721 | $Q_{76}$ | H | $CH_3$ | $OCHF_2$ | O | CH | |
| 2.722 | $Q_{76}$ | H | $CH_3$ | $OCH_3$ | O | N | |
| 2.723 | $Q_{76}$ | H | $OCH_3$ | $OCH_3$ | O | N | |
| 2.724 | $Q_{76}$ | H | $CH_3$ | $CH_3$ | O | N | |
| 2.725 | $Q_{76}$ | H | $CH_3$ | Cl | O | CH | |
| 2.726 | $Q_{76}$ | H | $OCH_3$ | Cl | O | CH | |
| 2.727 | $Q_{76}$ | H | $OC_2H_5$ | $CH_3$ | O | CH | |
| 2.728 | $Q_{76}$ | H | $OC_2H_5$ | $CH_3$ | O | N | |
| 2.729 | $Q_{76}$ | H | cyclopropyl | $OCH_3$ | O | CH | |
| 2.730 | $Q_{76}$ | H | cyclopropyl | $OCH_3$ | O | N | |
| 2.731 | $Q_{76}$ | H | $OCH_3$ | $N(CH_3)_2$ | O | N | |
| 2.732 | $Q_{76}$ | H | $CH_3$ | $C_2H_5$ | O | N | |
| 2.733 | $Q_{76}$ | H | $OCH_3$ | $C_2H_5$ | O | N | |
| 2.734 | $Q_{76}$ | H | $OCH_3$ | $CH_2OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.735 | $Q_{76}$ | H | $OCH_3$ | $OCHF_2$ | O | CH | |
| 2.736 | $Q_{76}$ | H | $OCH_3$ | $N(CH_3)_2$ | O | CH | |
| 2.737 | $Q_{76}$ | H | $OCH_3$ | $SCH_3$ | O | CH | |
| 2.738 | $Q_{76}$ | H | $OCH_3$ | $CH_2Cl$ | O | CH | |
| 2.739 | $Q_{77}$ | H | $CH_3$ | $CH_3$ | O | CH | |
| 2.740 | $Q_{77}$ | H | $OCH_3$ | $OCH_3$ | O | CH | |
| 2.741 | $Q_{77}$ | H | $CH_3$ | $OCH_3$ | O | CH | |
| 2.742 | $Q_{77}$ | H | $CH_3$ | $OCHF_2$ | O | CH | |
| 2.743 | $Q_{77}$ | H | $CH_3$ | $OCH_3$ | O | N | |
| 2.744 | $Q_{77}$ | H | $OCH_3$ | $OCH_3$ | O | N | |
| 2.745 | $Q_{77}$ | H | $CH_3$ | $CH_3$ | O | CH | |
| 2.746 | $Q_{77}$ | H | $OCHF_2$ | $OCHF_2$ | O | CH | |
| 2.747 | $Q_{77}$ | H | $CH_3$ | Cl | O | CH | |
| 2.748 | $Q_{77}$ | H | $OCH_3$ | Cl | O | CH | |
| 2.749 | $Q_{77}$ | H | $OC_2H_5$ | $C_2H_5$ | O | CH | |
| 2.750 | $Q_{77}$ | H | $OC_2H_5$ | $C_2H_5$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.751 | $Q_{77}$ | H | $OC_2H_5$ | $CH_3$ | O | N | |
| 2.752 | $Q_{77}$ | H | $OC_2H_5$ | $C_2H_5$ | O | N | |
| 2.753 | $Q_{77}$ | H | cyclopropyl | $OCH_3$ | O | CH | |
| 2.754 | $Q_{77}$ | H | cyclopropyl | $OCH_3$ | O | N | |
| 2.755 | $Q_{77}$ | $CH_3$ | $CH_3$ | $OCH_3$ | O | CH | |
| 2.756 | $Q_{77}$ | $CH_3$ | $CH_3$ | $OCH_3$ | O | N | |
| 2.757 | $Q_{77}$ | H | $CH_3$ | $OCH_3$ | S | CH | |
| 2.758 | $Q_{77}$ | H | $CH_3$ | $OCH_3$ | S | N | |
| 2.759 | $Q_{77}$ | $CH_3$ | $CH_3$ | $OCH_3$ | S | N | |
| 2.760 | $Q_{77}$ | $CH_3$ | $OCH_3$ | $OCH_3$ | O | N | |
| 2.761 | $Q_{77}$ | H | $OCH_3$ | $N(CH_3)_2$ | O | N | |
| 2.762 | $Q_{77}$ | H | $CH_3$ | $C_2H_5$ | O | N | |
| 2.763 | $Q_{77}$ | H | $OC_2H_5$ | $OC_2H_5$ | O | N | |
| 2.764 | $Q_{77}$ | H | $OC_2H_5$ | $OCH_3$ | O | N | |
| 2.765 | $Q_{77}$ | H | $OCH_3$ | $OCH(CH_3)_2$ | O | N | |
| 2.766 | $Q_{77}$ | H | $OCH_3$ | $SCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.767 | $Q_{77}$ | H | $OCH_3$ | $OC_2H_4OCH_3$ | O | N | |
| 2.768 | $Q_{77}$ | H | $CH_3$ | $CH_2Cl$ | O | N | |
| 2.769 | $Q_{77}$ | H | $OCH_3$ | $C_2H_5$ | O | N | |
| 2.770 | $Q_{77}$ | H | $OCH_3$ | $OCH_2CF_3$ | O | N | |
| 2.771 | $Q_{77}$ | H | $OCH_3$ | $CF_3$ | O | N | |
| 2.772 | $Q_{77}$ | H | $OCH_3$ | $CH_2OCH_3$ | O | CH | |
| 2.773 | $Q_{77}$ | H | $OCH_3$ | $OCHF_2$ | O | CH | |
| 2.774 | $Q_{77}$ | H | Cl | $OCHF_2$ | O | CH | |
| 2.775 | $Q_{77}$ | H | $OCHF_2$ | $N(CH_3)$ | O | CH | |
| 2.776 | $Q_{77}$ | H | $OC_2H_5$ | $OCHF_2$ | O | CH | |
| 2.777 | $Q_{77}$ | H | $OCHF_2$ | $CH_2F$ | O | N | |
| 2.778 | $Q_{77}$ | H | $OCH_3$ | $N(CH_3)_2$ | O | CH | |
| 2.779 | $Q_{77}$ | H | $OCH_3$ | $SCH_3$ | O | CH | |
| 2.780 | $Q_{77}$ | H | $OCH_3$ | $SCHF_2$ | O | CH | |
| 2.781 | $Q_{77}$ | H | $OCH_3$ | $CH_2CF_3$ | O | CH | |
| 2.782 | $Q_{77}$ | H | $OCH_3$ | $CH_2Cl$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.783 | $Q_{78}$ | H | $CH_3$ | $CH_3$ | O | CH | |
| 2.784 | $Q_{78}$ | H | $OCH_3$ | $CH_3$ | O | CH | |
| 2.785 | $Q_{78}$ | H | $CH_3$ | $OCH_3$ | O | CH | |
| 2.786 | $Q_{78}$ | H | $CH_3$ | $OCHF_2$ | O | CH | |
| 2.787 | $Q_{78}$ | H | $OCH_3$ | $CH_3$ | O | N | |
| 2.788 | $Q_{78}$ | H | $OCH_3$ | $OCH_3$ | O | N | |
| 2.789 | $Q_{78}$ | H | $CH_3$ | $CH_3$ | O | N | |
| 2.790 | $Q_{78}$ | H | $CH_3$ | Cl | O | CH | |
| 2.791 | $Q_{78}$ | H | $OCH_3$ | Cl | O | CH | |
| 2.792 | $Q_{78}$ | H | $OC_2H_5$ | $CH_3$ | O | CH | |
| 2.793 | $Q_{78}$ | H | $OC_2H_5$ | $CH_3$ | O | N | |
| 2.794 | $Q_{78}$ | H | cyclopropyl | $OCH_3$ | O | CH | |
| 2.795 | $Q_{78}$ | H | cyclopropyl | $OCH_3$ | O | N | |
| 2.796 | $Q_{78}$ | H | $OCH_3$ | $N(CH_3)_2$ | O | N | |
| 2.797 | $Q_{78}$ | H | $CH_3$ | $C_2H_5$ | O | N | |
| 2.798 | $Q_{78}$ | H | $OCH_3$ | $C_2H_5$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.799 | $Q_{78}$ | H | $OCH_3$ | $CH_2OCH_3$ | O | CH | |
| 2.800 | $Q_{78}$ | H | $OCH_3$ | $OCHF_2$ | O | CH | |
| 2.801 | $Q_{78}$ | H | $OCH_3$ | $N(CH_3)_2$ | O | CH. | |
| 2.802 | $Q_{78}$ | H | $OCH_3$ | $SCH_3$ | O | CH | |
| 2.803 | $Q_{78}$ | H | $OCH_3$ | $CH_2Cl$ | O | CH | |
| 2.804 | $Q_{79}$ | H | $CH_3$ | $CH_3$ | O | CH | |
| 2.805 | $Q_{79}$ | H | $CH_3$ | $OCH_3$ | O | CH | |
| 2.806 | $Q_{79}$ | H | $OCH_3$ | $OCH_3$ | O | CH | |
| 2.807 | $Q_{79}$ | H | $OCH_3$ | Cl | O | CH | |
| 2.808 | $Q_{79}$ | H | $CH_3$ | $OCHF_2$ | O | CH | |
| 2.809 | $Q_{79}$ | H | $CH_3$ | $CH_3$ | O | N | |
| 2.810 | $Q_{79}$ | H | $CH_3$ | $OCH_3$ | O | N | |
| 2.811 | $Q_{79}$ | H | $OCH_3$ | $OCH_3$ | O | N | |
| 2.812 | $Q_{79}$ | H | $OCH_3$ | $N(CH_3)_2$ | O | N | |
| 2.813 | $Q_{80}$ | H | $CH_3$ | $CH_3$ | O | CH | |
| 2.814 | $Q_{80}$ | H | $CH_3$ | $OCH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.815 | $Q_{80}$ | H | $OCH_3$ | $OCH_3$ | O | CH | |
| 2.816 | $Q_{80}$ | H | $OCH_3$ | Cl | O | CH | |
| 2.817 | $Q_{80}$ | H | $CH_3$ | $OCHF_2$ | O | CH | |
| 2.818 | $Q_{80}$ | H | $CH_3$ | $CH_3$ | O | N | |
| 2.819 | $Q_{80}$ | H | $CH_3$ | $OCH_3$ | O | N | |
| 2.820 | $Q_{80}$ | H | $OCH_3$ | $OCH_3$ | O | N | |
| 2.821 | $Q_{80}$ | H | $OCH_3$ | $N(CH_3)_2$ | O | N | |
| 2.822 | $Q_3$ | $CH_3$ | $OCHF_2$ | $CH_3$ | O | CH | |
| 2.823 | $Q_3$ | H | $OCHF_2$ | $OCHF_2$ | O | CH | |
| 2.824 | $Q_3$ | H | $OCHF_2$ | $CH_3$ | S | CH | |
| 2.825 | $Q_3$ | H | $OCHF_2$ | $NHCH_3$ | O | CH | |
| 2.826 | $Q_3$ | H | $OCHF_2$ | $N(CH_3)C_2H_5$ | O | CH | |
| 2.827 | $Q_3$ | H | $OCHF_2$ | $C_2H_5$ | O | CH | |
| 2.828 | $Q_3$ | H | $OCHF_2$ | $OCH_3$ | O | CH | |
| 2.829 | $Q_3$ | H | $OCHF_2$ | Cl | O | CH | |
| 2.830 | $Q_3$ | H | $OCHF_2$ | $CH_2F$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.831 | $Q_3$ | H | $OCHF_2$ | $CF_3$ | O | CH | |
| 2.832 | $Q_3$ | H | $OCHF_2$ | F | O | CH | |
| 2.833 | $Q_3$ | H | $OCHF_2$ | Br | O | CH | |
| 2.834 | $Q_3$ | H | $OCHF_2$ | $OCH_2CH_2F$ | O | CH | |
| 2.835 | $Q_3$ | H | $OCHF_2$ | $OCH_2CF_3$ | O | CH | |
| 2.836 | $Q_3$ | H | $OC_2H_5$ | $C_2H_5$ | O | CH | |
| 2.837 | $Q_3$ | H | $OC_2H_5$ | $OC_2H_5$ | O | CH | |
| 2.838 | $Q_3$ | H | $OC_2H_5$ | $C_2H_5$ | O | N | |
| 2.839 | $Q_3$ | H | $OC_2H_5$ | $OC_2H_5$ | O | N | |
| 2.840 | $Q_3$ | H | cyclopropyl | $OCH_3$ | O | CH | |
| 2.841 | $Q_3$ | H | cyclopropyl | $OCH_3$ | O | N | 190-192° |
| 2.842 | $Q_3$ | H | cyclopropyl | $OC_2H_5$ | O | CH | |
| 2.843 | $Q_3$ | H | $CH_2OC_2H_5$ | $OCH_3$ | O | N | |
| 2.844 | $Q_1$ | H | $OCHF_2$ | F | O | CH | |
| 2.845 | $Q_1$ | H | $OCHF_2$ | Br | O | CH | |
| 2.846 | $Q_1$ | H | $OCHF_2$ | $OCH_2CH_2F$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.847 | $Q_1$ | $CH_3$ | $OCHF_2$ | $OCH_2CF_3$ | O | CH | |
| 2.848 | $Q_1$ | H | $OCHF_2$ | $NHCH_3$ | O | CH | |
| 2.849 | $Q_1$ | H | $OC_2H_5$ | $C_2H_5$ | O | CH | |
| 2.850 | $Q_1$ | H | $OC_2H_5$ | $OC_2H_5$ | O | CH | |
| 2.851 | $Q_1$ | H | $OC_2H_5$ | $C_2H_5$ | O | N | |
| 2.852 | $Q_1$ | H | $OC_2H_5$ | $OC_2H_5$ | O | N | |
| 2.853 | $Q_1$ | H | cyclopropyl | $OCH_3$ | O | N | |
| 2.854 | $Q_1$ | H | cyclopropyl | $OCH_3$ | O | CH | |
| 2.855 | $Q_1$ | H | cyclopropyl | $OC_2H_5$ | O | CH | |
| 2.856 | $Q_1$ | H | $CH_2OC_2H_5$ | $OCH_3$ | O | CH | |
| 2.857 | $Q_3$ | H | $OCH_3$ | $C_2H_5$ | O | N | 186-188° |
| 2.858 | $Q_3$ | H | $CH_3$ | $C_2H_5$ | O | N | 195-198° |
| 2.859 | $Q_3$ | H | $CH_3$ | $OC_2H_5$ | O | N | 179-181° |
| 2.860 | $Q_{10}$ | H | Cl | $OCH_3$ | O | CH | 195-197° |
| 2.861 | $Q_{30}$ | H | $C_2H_5$ | $OC_2H_5$ | O | N | |
| 2.862 | $Q_{30}$ | H | $OCHF_2$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.863 | $Q_{30}$ | H | $OCH_3$ | cyclopropyl | O | N | |
| 2.864 | $Q_{30}$ | H | $OCH_3$ | cyclopropyl | O | CH | |
| 2.865 | $Q_{30}$ | H | $OCH_3$ | $OCHF_2$ | O | CH | |
| 2.866 | $Q_{30}$ | H | $OC_2H_5$ | $OC_2H_5$ | O | CH | |
| 2.867 | $Q_{30}$ | H | $CH_3$ | $OCHF_2$ | O | CH | |
| 3.868 | $Q_{31}$ | H | $C_2H_5$ | $OC_2H_5$ | O | N | |
| 2.869 | $Q_{31}$ | H | $OCHF_2$ | $OCHF_2$ | O | CH | |
| 2.870 | $Q_{31}$ | H | $OCH_3$ | cyclopropyl | O | CH | |
| 2.871 | $Q_{31}$ | H | $OCH_3$ | cyclopropyl | O | N | |
| 2.872 | $Q_{31}$ | H | $OCH_3$ | $OCHF_2$ | O | CH | |
| 2.873 | $Q_{31}$ | H | $OC_2H_5$ | $OC_2H_5$ | O | CH | |
| 2.874 | $Q_{31}$ | H | $CH_3$ | $OCHF_2$ | O | CH | |
| 2.875 | $Q_{32}$ | H | $C_2H_5$ | $OC_2H_5$ | O | N | |
| 2.876 | $Q_{32}$ | H | $OCHF_2$ | $OCHF_2$ | O | CH | |
| 2.877 | $Q_{32}$ | H | $OCHF_2$ | $OCH_3$ | O | CH | |
| 2.878 | $Q_{32}$ | H | $OCHF_2$ | $CH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.879 | $Q_{32}$ | H | $OCH_3$ | cyclopropyl | O | CH | |
| 2.880 | $Q_{32}$ | H | $OCH_3$ | cyclopropyl | O | N | |
| 2.881 | $Q_{32}$ | H | $OC_2H_5$ | $OC_2H_5$ | O | N | |
| 2.882 | $Q_{32}$ | H | $OC_2H_5$ | $OC_2H_5$ | O | CH | |
| 2.883 | $Q_{33}$ | H | $C_2H_5$ | $OC_2H_5$ | O | N | |
| 2.884 | $Q_{33}$ | H | $OCHF_2$ | $OCHF_2$ | O | CH | |
| 2.885 | $Q_{33}$ | H | $OCHF_2$ | $OCH_3$ | O | CH | |
| 2.886 | $Q_{33}$ | H | $OCHF_2$ | $CH_3$ | O | CH | |
| 2.887 | $Q_{33}$ | H | $OCH_3$ | cyclopropyl | O | CH | |
| 2.888 | $Q_{33}$ | H | $OCH_3$ | cyclopropyl | O | N | |
| 2.889 | $Q_{33}$ | H | $OC_2H_5$ | $OC_2H_5$ | O | N | |
| 2.890 | $Q_{33}$ | H | $OC_2H_5$ | $OC_2H_5$ | O | CH | |
| 2.891 | $Q_{34}$ | H | $C_2H_5$ | $OC_2H_5$ | O | N | |
| 2.892 | $Q_{34}$ | H | $OCHF_2$ | $OCHF_2$ | O | CH | |
| 2.893 | $Q_{34}$ | H | $OCHF_2$ | $OCH_3$ | O | CH | |
| 2.894 | $Q_{34}$ | H | $OCHF_2$ | $CH_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.895 | $Q_{34}$ | H | $OCH_3$ | cyclopropyl | O | CH | |
| 2.896 | $Q_{34}$ | H | $OCH_3$ | cyclopropyl | O | N | |
| 2.897 | $Q_{34}$ | H | $OC_2H_5$ | $OC_2H_5$ | O | N | |
| 2.898 | $Q_{34}$ | H | $OC_2H_5$ | $OC_2H_5$ | O | CH | |
| 2.899 | $Q_{35}$ | H | $OCHF_2$ | $OCHF_2$ | O | CH | |
| 2.900 | $Q_{35}$ | H | $OCHF_2$ | $OCH_3$ | O | CH | |
| 2.901 | $Q_{35}$ | H | $OCHF_2$ | $CH_3$ | O | CH | |
| 2.902 | $Q_{35}$ | H | $OCH_3$ | cyclopropyl | O | CH | |
| 2.903 | $Q_{35}$ | H | $OCH_3$ | cyclopropyl | O | N | |
| 2.904 | $Q_{35}$ | H | $OC_2H_5$ | $C_2H_5$ | O | N | |
| 2.905 | $Q_{35}$ | H | $OC_2H_5$ | $OC_2H_5$ | O | CH | |
| 2.906 | $Q_{35}$ | H | $OC_2H_5$ | $OC_2H_5$ | O | N | |
| 2.907 | $Q_{36}$ | H | $OCHF_2$ | $OCHF_2$ | O | CH | |
| 2.908 | $Q_{36}$ | H | $OCHF_2$ | $OCH_3$ | O | CH | |
| 2.909 | $Q_{36}$ | H | $OCHF_2$ | $CH_3$ | O | CH | |
| 2.910 | $Q_{36}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.911 | $Q_{36}$ | H | $OCH_3$ | cyclopropyl | O | N | |
| 2.912 | $Q_{36}$ | H | $OC_2H_5$ | $C_2H_5$ | O | N | |
| 2.913 | $Q_{36}$ | H | $OC_2H_5$ | $OC_2H_5$ | O | N | |
| 2.914 | $Q_{36}$ | H | $OC_2H_5$ | $OC_2H_5$ | O | N | |
| 2.915 | $Q_{39}$ | H | $OCHF_2$ | $OCHF_2$ | O | CH | |
| 2.916 | $Q_{39}$ | H | $OCHF_2$ | $OCH_3$ | O | CH | |
| 2.917 | $Q_{39}$ | H | $OCHF_2$ | $CH_3$ | O | CH | |
| 2.918 | $Q_{39}$ | H | $OCH_3$ | cyclopropyl | O | CH | |
| 2.919 | $Q_{39}$ | H | $OCH_3$ | cyclopropyl | O | N | |
| 2.920 | $Q_{39}$ | H | $OC_2H_5$ | $C_2H_5$ | O | N | |
| 2.921 | $Q_{39}$ | H | $OC_2H_5$ | $OC_2H_5$ | O | N | |
| 2.922 | $Q_{39}$ | H | $OC_2H_5$ | $OC_2H_5$ | O | CH | |
| 2.923 | $Q_{40}$ | H | $OCHF_2$ | $OCHF_2$ | O | CH | |
| 2.924 | $Q_{40}$ | H | $OCHF_2$ | $OCH_3$ | O | CH | |
| 2.925 | $Q_{40}$ | H | $OCHF_2$ | $CH_3$ | O | CH | |
| 2.926 | $Q_{40}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.927 | $Q_{40}$ | H | $OCH_3$ | cyclopropyl | O | N | |
| 2.928 | $Q_{40}$ | H | $OC_2H_5$ | $C_2H_5$ | O | N | |
| 2.929 | $Q_{40}$ | H | $OC_2H_5$ | $OC_2H_5$ | O | N | |
| 2.930 | $Q_{40}$ | H | $OC_2H_5$ | $OC_2H_5$ | O | CH | |
| 2.931 | $Q_{41}$ | H | $OCHF_2$ | $OCHF_2$ | O | CH | |
| 2.932 | $Q_{41}$ | H | $OCHF_2$ | $OCH_3$ | O | CH | |
| 2.933 | $Q_{41}$ | H | $OCHF_2$ | $CH_3$ | O | CH | |
| 2.934 | $Q_{41}$ | H | $OCH_3$ | cyclopropyl | O | CH | |
| 2.935 | $Q_{41}$ | H | $OCH_3$ | cyclopropyl | O | N | |
| 2.936 | $Q_{41}$ | H | $OC_2H_5$ | $C_2H_5$ | O | N | |
| 2.937 | $Q_{41}$ | H | $OC_2H_5$ | $OC_2H_5$ | O | N | |
| 2.938 | $Q_{41}$ | H | $OC_2H_5$ | $OC_2H_5$ | O | CH | |
| 2.939 | $Q_{42}$ | H | $OCHF_2$ | $OCHF_2$ | O | CH | |
| 2.940 | $Q_{42}$ | H | $OCHF_2$ | $OCH_3$ | O | CH | |
| 2.941 | $Q_{42}$ | H | $OCHF_2$ | $CH_3$ | O | CH | |
| 2.942 | $Q_{42}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.943 | $Q_{42}$ | H | $OCH_3$ | cyclopropyl | O | N | |
| 2.944 | $Q_{42}$ | H | $OC_2H_5$ | $C_2H_5$ | O | N | |
| 2.945 | $Q_{42}$ | H | $OC_2H_5$ | $OC_2H_5$ | O | N | |
| 2.946 | $Q_{42}$ | H | $OC_2H_5$ | $OC_2H_5$ | O | CH | |
| 2.947 | $Q_{43}$ | H | $OCHF_2$ | $OCHF_2$ | O | CH | |
| 2.948 | $Q_{43}$ | H | $OCHF_2$ | $OCH_3$ | O | CH | |
| 2.949 | $Q_{43}$ | H | $OCHF_2$ | $CH_3$ | O | CH | |
| 2.950 | $Q_{43}$ | H | $OCH_3$ | cyclopropyl | O | CH | |
| 2.951 | $Q_{43}$ | H | $OCH_3$ | cyclopropyl | O | N | |
| 2.952 | $Q_{43}$ | H | $OC_2H_5$ | $C_2H_5$ | O | N | |
| 2.953 | $Q_{43}$ | H | $OC_2H_5$ | $OC_2H_5$ | O | N | |
| 2.954 | $Q_{43}$ | H | $OC_2H_5$ | $OC_2H_5$ | O | N | |
| 2.955 | $Q_{44}$ | H | $OCHF_2$ | $OCHF_2$ | O | CH | |
| 2.956 | $Q_{44}$ | H | $OCHF_2$ | $OCH_3$ | O | CH | |
| 2.957 | $Q_{44}$ | H | $OCHF_2$ | $CH_3$ | O | CH | |
| 2.958 | $Q_{44}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.959 | $Q_{44}$ | H | $OCH_3$ | cyclopropyl | O | N | |
| 2.960 | $Q_{44}$ | H | $OC_2H_5$ | $C_2H_5$ | O | N | |
| 2.961 | $Q_{44}$ | H | $OC_2H_5$ | $OC_2H_5$ | O | N | |
| 2.962 | $Q_{44}$ | H | $OC_2H_5$ | $OC_2H_5$ | O | CH | |
| 2.963 | $Q_{45}$ | H | $OCHF_2$ | $OCHF_2$ | O | CH | |
| 2.964 | $Q_{45}$ | H | $OCHF_2$ | $OCH_3$ | O | CH | |
| 2.965 | $Q_{45}$ | H | $OCHF_2$ | $CH_3$ | O | CH | |
| 2.966 | $Q_{45}$ | H | $OCH_3$ | cyclopropyl | O | CH | |
| 2.967 | $Q_{45}$ | H | $OCH_3$ | cyclopropyl | O | N | |
| 2.968 | $Q_{45}$ | H | $OC_2H_5$ | $C_2H_5$ | O | N | |
| 2.969 | $Q_{45}$ | H | $OC_2H_5$ | $OC_2H_5$ | O | N | |
| 2.970 | $Q_{45}$ | H | $OC_2H_5$ | $OC_2H_5$ | O | CH | |
| 2.971 | $Q_{46}$ | H | $OCHF_2$ | $OCHF_2$ | O | CH | |
| 2.972 | $Q_{46}$ | H | $OCHF_2$ | $OCH_3$ | O | CH | |
| 2.973 | $Q_{46}$ | H | $OCHF_2$ | $CH_3$ | O | CH | |
| 2.974 | $Q_{46}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.975 | $Q_{46}$ | H | $OCH_3$ | cyclopropyl | O | N | |
| 2.976 | $Q_{46}$ | H | $OC_2H_5$ | $C_2H_5$ | O | N | |
| 2.977 | $Q_{46}$ | H | $OC_2H_5$ | $OC_2H_5$ | O | N | |
| 2.978 | $Q_{46}$ | H | $OC_2H_5$ | $OC_2H_5$ | O | CH | |
| 2.979 | $Q_{47}$ | H | $OCHF_2$ | $OCHF_2$ | O | CH | |
| 2.980 | $Q_{47}$ | H | $OCHF_2$ | $OCH_3$ | O | CH | |
| 2.981 | $Q_{47}$ | H | $OCHF_2$ | $CH_3$ | O | CH | |
| 2.982 | $Q_{47}$ | H | $OCH_3$ | cyclopropyl | O | CH | |
| 2.983 | $Q_{47}$ | H | $OCH_3$ | cyclopropyl | O | N | |
| 2.984 | $Q_{47}$ | H | $OC_2H_5$ | $C_2H_5$ | O | N | |
| 2.985 | $Q_{47}$ | H | $OC_2H_5$ | $OC_2H_5$ | O | N | |
| 2.986 | $Q_{47}$ | H | $OC_2H_5$ | $OC_2H_5$ | O | CH | |
| 2.987 | $Q_{48}$ | H | $OCHF_2$ | $OCHF_2$ | O | CH | |
| 2.988 | $Q_{48}$ | H | $OCHF_2$ | $OCH_3$ | O | CH | |
| 2.989 | $Q_{48}$ | H | $OCHF_2$ | $CH_3$ | O | CH | |
| 2.990 | $Q_{48}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.991 | $Q_{48}$ | H | $OCH_3$ | cyclopropyl | O | N | |
| 2.992 | $Q_{48}$ | H | $OC_2H_5$ | $C_2H_5$ | O | N | |
| 2.993 | $Q_{48}$ | H | $OC_2H_5$ | $OC_2H_5$ | O | N | |
| 2.994 | $Q_{48}$ | H | $OC_2H_5$ | $OC_2H_5$ | O | CH | |
| 2.995 | $Q_{49}$ | H | $CH_3$ | $OCH_3$ | O | N | 178-179° |
| 2.996 | $Q_{49}$ | H | $CH_3$ | $OCH_3$ | O | CH | 201-202° |
| 2.997 | $Q_{49}$ | H | $OCH_3$ | $OCH_3$ | O | CH | 216-217° |
| 2.998 | $Q_{49}$ | H | $CH_3$ | $OCHF_2$ | O | CH | 189-190° |
| 2.999 | $Q_{49}$ | H | $OCH_3$ | Cl | O | CH | 216-217° |
| 2.1000 | $Q_{49}$ | H | $OCHF_2$ | $OCHF_2$ | O | CH | |
| 2.1001 | $Q_{49}$ | H | $OCHF_2$ | $N(CH_3)_2$ | O | CH | |
| 2.1002 | $Q_{49}$ | H | $OCHF_2$ | $C_2H_5$ | O | CH | |
| 2.1003 | $Q_{49}$ | H | $OCHF_2$ | $OCH_3$ | O | CH | |
| 2.1004 | $Q_{49}$ | H | $OCHF_2$ | Cl | O | CH | |
| 2.1005 | $Q_{49}$ | H | $OCHF_2$ | $CH_2F$ | O | CH | |
| 2.1006 | $Q_{49}$ | H | $OCHF_2$ | $CF_3$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1007 | $Q_{49}$ | H | $OCHF_2$ | F | O | CH | |
| 2.1008 | $Q_{49}$ | H | $OCHF_2$ | Br | O | CH | |
| 2.1009 | $Q_{49}$ | H | $OCHF_2$ | $OCH_2CH_2F$ | O | CH | |
| 2.1010 | $Q_{49}$ | H | $OCHF_2$ | $OCH_2CF_3$ | O | CH | |
| 2.1011 | $Q_{49}$ | H | $OC_2H_5$ | $C_2H_5$ | O | CH | |
| 2.1012 | $Q_{49}$ | H | $OC_2H_5$ | $OC_2H_5$ | O | CH | |
| 2.1013 | $Q_{49}$ | H | $OC_2H_5$ | $C_2H_5$ | O | N | |
| 2.1014 | $Q_{49}$ | H | $OC_2H_5$ | $OC_2H_5$ | O | N | |
| 2.1015 | $Q_{49}$ | H | cyclopropyl | $OCH_3$ | O | N | |
| 2.1016 | $Q_{49}$ | H | cyclopropyl | $OCH_3$ | O | CH | |
| 2.1017 | $Q_{49}$ | H | cyclopropyl | $OC_2H_5$ | O | CH | |
| 2.1018 | $Q_{49}$ | H | $CH_2OC_2H_5$ | $OCH_3$ | O | CH | |
| 2.1019 | $Q_{50}$ | H | $CH_3$ | $CHF_2$ | O | CH | 174° |
| 2.1020 | $Q_{50}$ | H | $OCH_3$ | Cl | O | CH | 201-203° |
| 2.1021 | $Q_{50}$ | H | $OCHF_2$ | $OCHF_2$ | O | CH | |
| 2.1022 | $Q_{50}$ | $CH_3$ | $OCHF_2$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1023 | $Q_{50}$ | H | $OCHF_2$ | $N(CH_3)_2$ | O | CH | |
| 2.1024 | $Q_{50}$ | H | $OCHF_2$ | $N(CH_3)C_2H_5$ | O | CH | |
| 2.1025 | $Q_{50}$ | H | $OCHF_2$ | $C_2H_5$ | O | CH | |
| 2.1026 | $Q_{50}$ | H | $OCHF_2$ | $OCH_3$ | O | CH | |
| 2.1027 | $Q_{50}$ | H | $OCHF_2$ | Cl | O | CH | |
| 2.1028 | $Q_{50}$ | H | $OCHF_2$ | $CH_2F$ | O | CH | |
| 2.1029 | $Q_{50}$ | H | $OCHF_2$ | $CF_3$ | O | CH | |
| 2.1030 | $Q_{50}$ | H | $OCHF_2$ | F | O | CH | |
| 2.1031 | $Q_{50}$ | H | $OCHF_2$ | Br | O | CH | |
| 2.1032 | $Q_{50}$ | H | $OCHF_2$ | $OCH_2CH_2F$ | O | CH | |
| 2.1033 | $Q_{50}$ | H | $OCHF_2$ | $OCH_2CF_3$ | O | CH | |
| 2.1034 | $Q_{50}$ | H | $OCHF_2$ | $NHCH_3$ | O | CH | |
| 2.1035 | $Q_{50}$ | H | $OC_2H_5$ | $C_2H_5$ | O | CH | |
| 2.1036 | $Q_{50}$ | H | $OC_2H_5$ | $OC_2H_5$ | O | CH | |
| 2.1037 | $Q_{50}$ | H | $OC_2H_5$ | $C_2H_5$ | O | N | |
| 2.1038 | $Q_{50}$ | H | $OC_2H_5$ | $OC_2H_5$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1039 | $Q_{50}$ | H | $OCH_3$ | cyclopropyl | O | N | |
| 2.1040 | $Q_{50}$ | H | $OCH_3$ | cyclopropyl | O | CH | |
| 2.1041 | $Q_{50}$ | H | $OC_2H_5$ | cyclopropyl | O | CH. | |
| 2.1042 | $Q_{50}$ | H | $OCH_3$ | $CH_2OC_2H_5$ | O | CH | |
| 2.1043 | $Q_{51}$ | H | $OCHF_2$ | $OCHF_2$ | O | CH | |
| 2.1044 | $Q_{51}$ | H | $OCHF_2$ | $OCH_3$ | O | CH | |
| 2.1045 | $Q_{51}$ | H | $OCHF_2$ | $CH_3$ | O | CH | |
| 2.1046 | $Q_{51}$ | H | $OCH_3$ | cyclopropyl | O | CH | |
| 2.1047 | $Q_{51}$ | H | $OCH_3$ | cyclopropyl | O | N | |
| 2.1048 | $Q_{51}$ | H | $OC_2H_5$ | $C_2H_5$ | O | N | |
| 2.1049 | $Q_{51}$ | H | $OC_2H_5$ | $OC_2H_5$ | O | N | |
| 2.1050 | $Q_{51}$ | H | $OC_2H_5$ | $OC_2H_5$ | O | CH | |
| 2.1051 | $Q_{52}$ | H | $OCHF_2$ | $OCHF_2$ | O | CH | |
| 2.1052 | $Q_{52}$ | H | $OCHF_2$ | $OCH_3$ | O | CH | |
| 2.1053 | $Q_{52}$ | H | $OCHF_2$ | $CH_3$ | O | CH | |
| 2.1054 | $Q_{52}$ | H | $OCH_3$ | cyclopropyl | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1055 | $Q_{52}$ | H | $OCH_3$ | cyclopropyl | O | N | |
| 2.1056 | $Q_{52}$ | H | $OC_2H_5$ | $C_2H_5$ | O | N | |
| 2.1057 | $Q_{52}$ | H | $OC_2H_5$ | $OC_2H_5$ | O | N | |
| 2.1058 | $Q_{52}$ | H | $OC_2H_5$ | $OC_2H_5$ | O | CH | |
| 2.1059 | $Q_{81}$ | H | $CH_3$ | $CH_3$ | O | CH | 214-215° |
| 2.1060 | $Q_{81}$ | H | $CH_3$ | $OCH_3$ | O | CH | 220-221° |
| 2.1061 | $Q_{81}$ | H | $CH_3$ | $OCH_3$ | O | N | 200-202° |
| 2.1062 | $Q_{81}$ | H | $OCHF_2$ | $OCHF_2$ | O | CH | |
| 2.1063 | $Q_{81}$ | H | $OCHF_2$ | $OCH_3$ | O | CH | |
| 2.1064 | $Q_{81}$ | H | $OCHF_2$ | $CH_3$ | O | CH | |
| 2.1065 | $Q_{81}$ | H | $OCH_3$ | cyclopropyl | O | CH | |
| 2.1066 | $Q_{81}$ | H | $OCH_3$ | cyclopropyl | O | N | |
| 2.1067 | $Q_{81}$ | H | $OC_2H_5$ | $C_2H_5$ | O | N | |
| 2.1068 | $Q_{81}$ | H | $OC_2H_5$ | $OC_2H_5$ | O | N | |
| 2.1069 | $Q_{81}$ | H | $OC_2H_5$ | $OC_2H_5$ | O | CH | |
| 2.1070 | $Q_{82}$ | H | $CH_3$ | $OCH_3$ | O | N | 196-197° |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1071 | $Q_{82}$ | H | $CH_3$ | $CH_3$ | O | CH | 200-201° |
| 2.1072 | $Q_{82}$ | H | $CH_3$ | $OCH_3$ | O | CH | 187-188° |
| 2.1073 | $Q_{82}$ | H | $OCH_3$ | $OCH_3$ | O | CH | 234-235° |
| 2.1074 | $Q_{82}$ | H | $OCHF_2$ | $CH_3$ | O | CH | |
| 2.1075 | $Q_{82}$ | H | $OCHF_2$ | $OCHF_2$ | O | CH | |
| 2.1076 | $Q_{82}$ | $CH_3$ | $OCHF_2$ | $OCHF_2$ | O | CH | |
| 2.1077 | $Q_{82}$ | H | $OCHF_2$ | $N(CH_3)_2$ | O | CH | |
| 2.1078 | $Q_{82}$ | H | $OCHF_2$ | $N(CH_3)C_2H_5$ | O | CH | |
| 2.1079 | $Q_{82}$ | H | $OCHF_2$ | $C_2H_5$ | O | CH | |
| 2.1080 | $Q_{82}$ | H | $OCHF_2$ | $OCH_3$ | O | CH | |
| 2.1081 | $Q_{82}$ | H | $OCHF_2$ | Cl | O | CH | |
| 2.1082 | $Q_{82}$ | H | $OCHF_2$ | $CH_2F$ | O | CH | |
| 2.1083 | $Q_{82}$ | H | $OCHF_2$ | $CF_3$ | O | CH | |
| 2.1084 | $Q_{82}$ | H | $OCHF_2$ | F | O | CH | |
| 2.1085 | $Q_{82}$ | H | $OCHF_2$ | Br | O | CH | |
| 2.1086 | $Q_{82}$ | H | $OCHF_2$ | $OCH_2CH_2F$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1087 | $Q_{82}$ | H | $OCHF_2$ | $OCH_2CF_3$ | O | CH | |
| 2.1088 | $Q_{82}$ | H | $OCHF_2$ | $NHCH_3$ | O | CH | |
| 2.1089 | $Q_{82}$ | H | $OC_2H_5$ | $C_2H_5$ | O | CH | |
| 2.1090 | $Q_{82}$ | H | $OC_2H_5$ | $OC_2H_5$ | O | CH | |
| 2.1091 | $Q_{82}$ | H | $OC_2H_5$ | $C_2H_5$ | O | N | |
| 2.1092 | $Q_{82}$ | H | $OC_2H_5$ | $OC_2H_5$ | O | N | |
| 2.1093 | $Q_{82}$ | H | $OCH_3$ | cyclopropyl | O | CH | |
| 2.1094 | $Q_{82}$ | H | $OCH_3$ | cyclopropyl | O | N | |
| 2.1095 | $Q_{82}$ | H | $OC_2H_5$ | cyclopropyl | O | CH | |
| 2.1096 | $Q_{82}$ | H | $OCH_3$ | $CH_2OC_2H_5$ | O | CH | |
| 2.1097 | $Q_{83}$ | H | $CH_3$ | $CH_3$ | O | CH | |
| 2.1098 | $Q_{83}$ | H | $OCH_3$ | $OCH_3$ | O | CH | |
| 2.1099 | $Q_{83}$ | H | $CH_3$ | $OCH_3$ | O | CH | |
| 2.1100 | $Q_{83}$ | H | $CH_3$ | $OCHF_2$ | O | CH | |
| 2.1101 | $Q_{83}$ | H | $CH_3$ | $OCH_3$ | O | N | |
| 2.1102 | $Q_{83}$ | H | $OCH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1103 | $Q_{83}$ | H | $CH_3$ | $CH_3$ | O | N | |
| 2.1104 | $Q_{83}$ | H | $OCHF_2$ | $OCHF_2$ | O | N | |
| 2.1105 | $Q_{83}$ | H | $CH_3$ | Cl | O | CH | |
| 2.1106 | $Q_{83}$ | H | $OCH_3$ | Cl | O | CH | |
| 2.1107 | $Q_{83}$ | H | $OC_2H_5$ | $OC_2H_5$ | O | CH | |
| 2.1108 | $Q_{83}$ | H | $OC_2H_5$ | $CH_3$ | O | CH | |
| 2.1109 | $Q_{83}$ | H | $OC_2H_5$ | $CH_3$ | O | N | |
| 2.1110 | $Q_{83}$ | H | $OC_2H_5$ | $CH_3$ | O | N | |
| 2.1111 | $Q_{83}$ | H | cyclopropyl | $OCH_3$ | O | CH | |
| 2.1112 | $Q_{83}$ | H | cyclopropyl | $OCH_3$ | O | N | |
| 2.1113 | $Q_{83}$ | $CH_3$ | $CH_3$ | $OCH_3$ | O | CH | |
| 2.1114 | $Q_{83}$ | $CH_3$ | $CH_3$ | $OCH_3$ | O | N | |
| 2.1115 | $Q_{83}$ | H | $CH_3$ | $OCH_3$ | S | CH | |
| 2.1116 | $Q_{83}$ | H | $CH_3$ | $OCH_3$ | S | N | |
| 2.1117 | $Q_{83}$ | $CH_3$ | $CH_3$ | $OCH_3$ | S | N | |
| 2.1118 | $Q_{83}$ | $CH_3$ | $OCH_3$ | $OCH_3$ | O | N | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1119 | $Q_{83}$ | H | $OCH_3$ | $N(CH_3)_2$ | O | N | |
| 2.1120 | $Q_{83}$ | H | $CH_3$ | $C_2H_5$ | O | N | |
| 2.1121 | $Q_{83}$ | H | $OC_2H_5$ | $OC_2H_5$ | O | N | |
| 2.1122 | $Q_{83}$ | H | $OC_2H_5$ | $OCH_3$ | O | N | |
| 2.1123 | $Q_{83}$ | H | $OCH_3$ | $OCH(CH_3)_2$ | O | N | |
| 2.1124 | $Q_{83}$ | H | $OCH_3$ | $SCH_3$ | O | N | |
| 2.1125 | $Q_{83}$ | H | $OCH_3$ | $OC_2H_4OCH_3$ | O | N | |
| 2.1126 | $Q_{83}$ | H | $CH_3$ | $CH_2Cl$ | O | N | |
| 2.1127 | $Q_{83}$ | H | $OCH_3$ | $C_2H_5$ | O | N | |
| 2.1128 | $Q_{83}$ | H | $OCH_3$ | $OCH_2CF_3$ | O | N | |
| 2.1129 | $Q_{83}$ | H | $OCH_3$ | $CF_3$ | O | N | |
| 2.1130 | $Q_{83}$ | H | $OCH_3$ | $CH_2OCH_3$ | O | CH | |
| 2.1131 | $Q_{83}$ | H | $OCH_3$ | $OCHF_2$ | O | CH | |
| 2.1132 | $Q_{83}$ | H | Cl | $OCHF_2$ | O | CH | |
| 2.1133 | $Q_{83}$ | H | $OCHF_2$ | $N(CH_3)_2$ | O | CH | |
| 2.1134 | $Q_{83}$ | H | $OC_2H_5$ | $OCHF_2$ | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1135 | $Q_{83}$ | H | $OCHF_2$ | $OCH_2F$ | O | CH | |
| 2.1136 | $Q_{83}$ | H | $OCH_3$ | $N(CH_3)_2$ | O | CH | |
| 2.1137 | $Q_{83}$ | H | $OCH_3$ | $SCH_3$ | O | CH | |
| 2.1138 | $Q_{83}$ | H | $OCH_3$ | $SCHF_2$ | O | CH | |
| 2.1139 | $Q_{83}$ | H | $OCH_3$ | $CH_2CF_3$ | O | CH | |
| 2.1140 | $Q_{83}$ | H | $OCH_3$ | $CH_2Cl$ | O | CH | |
| 2.1141 | $Q_{84}$ | H | $OCHF_2$ | $CH_3$ | O | CH | |
| 2.1142 | $Q_{84}$ | H | $OCHF_2$ | $OCHF_2$ | O | CH | |
| 2.1143 | $Q_{84}$ | H | $OCHF_2$ | $N(CH_3)_2$ | O | CH | |
| 2.1144 | $Q_{84}$ | H | $OCHF_2$ | $C_2H_5$ | O | CH | |
| 2.1145 | $Q_{84}$ | H | $OCHF_2$ | $OCH_3$ | O | CH | |
| 2.1146 | $Q_{84}$ | H | $OCHF_2$ | Cl | O | CH | |
| 2.1147 | $Q_{84}$ | H | $OCHF_2$ | $CH_2F$ | O | CH | |
| 2.1148 | $Q_{84}$ | H | $OCHF_2$ | $CF_3$ | O | CH | |
| 2.1149 | $Q_{84}$ | H | $OCHF_2$ | F | O | CH | |
| 2.1150 | $Q_{84}$ | H | $OCHF_2$ | Br | O | CH | |

Tabelle 2 (Fortsetzung)

| Verb. Nr. | Q | $R_2$ | $R_3$ | $R_4$ | Z | E | Smp. [°C] |
|---|---|---|---|---|---|---|---|
| 2.1151 | $Q_{84}$ | H | $OCHF_2$ | $OCH_2CH_2F$ | O | CH | |
| 2.1152 | $Q_{84}$ | H | $OCHF_2$ | $OCH_2CF_2$ | O | CH | |
| 2.1153 | $Q_{84}$ | $CH_3$ | $OCHF_2$ | $OCHF_2$ | O | CH. | |
| 2.1154 | $Q_{84}$ | H | $OC_2H_5$ | $C_2H_5$ | O | CH | |
| 2.1155 | $Q_{84}$ | H | $OC_2H_5$ | $OC_2H_5$ | O | CH | |
| 2.1156 | $Q_{84}$ | H | $OC_2H_5$ | $C_2H_5$ | O | N | |
| 2.1157 | $Q_{84}$ | H | $OC_2H_5$ | $OC_2H_5$ | O | N | |
| 2.1158 | $Q_{84}$ | H | $OCH_3$ | cyclopropyl | O | N | |
| 2.1159 | $Q_{84}$ | H | $OCH_3$ | cyclopropyl | O | CH | |
| 2.1160 | $Q_{84}$ | H | $OC_2H_5$ | cyclopropyl | O | CH | |
| 2.1161 | $Q_{84}$ | H | $OCH_3$ | $CH_2OC_2H_5$ | O | CH | |
| 2.1162 | $Q_3$ | H | $CF_3$ | $OCH_3$ | O | N | 172-175° |

Formulierungsbeispiele

Beispiel 3: Formulierungsbeispiele für Wirkstoffe der Formel I (% = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykolether (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10 % | 1 % |
| Octylphenolpolyethylenglykolether (4-5 Mol AeO) | 3 % | 3 % |
| Ca-Dodecylbenzolsulfonat | 3 % | 3 % |
| Ricinusölpolyglykolether (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 2 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Ethylenglykol | 10 % | 10 % |
| Nonylphenolpolyethylenglykolether (15 Mol AeO) | 6 % | 1 % |
| Na-Ligninsulfonat | 10 % | 5 % |
| Carboxymethylcellulose | 1 % | 1 % |

| | | |
|---|---|---|
| 37%ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

Biologische Beispiele

Beispiel 4: Herbizidwirkung vor dem Auflaufen der Pflanzen

Kunststofftöpfe werden mit expandiertem Vermiculit (Dichte: 0,135 $g/cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät. Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 6 Tagen werden die Töpfe zur Erhöhung der örtlichen Luftfeuchtigkeit mit lichtdurchlässigem Material abgedeckt und mit deionisiertem Wasser begossen. Nach dem 5. Tag wird dem Giesswasser 0,5% eines handelsüblichen Flüssigdüngers (®Greenzit) zugesetzt.
12 Tage nach der Aussaat wird der Versuch ausgewertet und die Wirkung auf die Versuchspflanzen nach dem folgendem Massstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze / Wirkstoff Nr. | Nasturtium | Stellaria | Agrostis | Digitaria |
|---|---|---|---|---|
| 2.0034 | 2 | 2 | 2 | 2 |
| 2.0035 | 2 | 2 | 2 | 2 |
| 2.0036 | 2 | 1 | 2 | 2 |
| 2.0037 | 2 | 3 | 3 | 4 |
| 2.0038 | 2 | 2 | 2 | 2 |
| 2.0039 | 1 | 2 | 1 | 2 |
| 2.0040 | 1 | 1 | 1 | 1 |
| 2.0041 | 2 | 3 | 2 | 3 |
| 2.0042 | 2 | 2 | 2 | 2 |
| 2.0043 | 1 | 3 | 1 | 3 |
| 2.0044 | 1 | 1 | 1 | 1 |
| 2.0045 | 2 | 2 | 1 | 2 |
| 2.0066 | 2 | 2 | 2 | 2 |
| 2.0070 | 2 | 2 | 1 | 2 |
| 2.0071 | 1 | 2 | 1 | 2 |
| 2.0072 | 2 | 2 | 1 | 2 |
| 2.0090 | 1 | 2 | 1 | 2 |
| 2.0094 | 1 | 1 | 1 | 1 |
| 2.0095 | 1 | 1 | 1 | 1 |
| 2.0096 | 1 | 1 | 1 | 1 |
| 2.0097 | 2 | 1 | 1 | 2 |

| Testpflanze / Wirkstoff Nr. | Nasturtium | Stellaria | Agrostis | Digitaria |
|---|---|---|---|---|
| 2.0119 | 2 | 2 | 1 | 4 |
| 2.0122 | 1 | 2 | 1 | 2 |
| 2.0123 | 1 | 2 | 1 | 4 |
| 2.0126 | 2 | 2 | 1 | 2 |
| 2.0127 | 1 | 1 | 1 | 1 |
| 2.0128 | 1 | 2 | 1 | 2 |
| 2.0250 | 2 | 2 | 1 | 1 |
| 2.0251 | 2 | 2 | 1 | 2 |
| 2.0252 | 2 | 2 | 1 | 2 |
| 2.0253 | 1 | 2 | 1 | 2 |
| 2.0254 | 1 | 1 | 1 | 1 |
| 2.0271 | 1 | 1 | 1 | 2 |
| 2.0274 | 1 | 2 | 1 | 2 |
| 2.0279 | 1 | 2 | 1 | 2 |
| 2.0433 | 2 | 2 | 2 | 2 |
| 2.0435 | 1 | 2 | 1 | 3 |
| 2.0437 | 2 | 2 | 3 | 4 |
| 2.0565 | 2 | 2 | 2 | 2 |
| 2.0567 | 1 | 2 | 1 | 2 |
| 2.0568 | 2 | 7 | 2 | 7 |
| 2.0569 | 2 | 2 | 2 | 4 |
| 2.0574 | 1 | 2 | 1 | 3 |
| 2.0840 | 2 | 1 | 3 | 2 |
| 2.0857 | 2 | 2 | 2 | 3 |
| 2.0858 | 2 | 3 | 3 | 5 |
| 2.0859 | 2 | 2 | 2 | 2 |

| Testpflanze / Wirkstoff Nr. | Nasturtium | Stellaria | Agrostis | Digitaria |
|---|---|---|---|---|
| 2.0860 | 1 | 1 | 1 | 2 |
| 2.0995 | 2 | 2 | 1 | 2 |
| 2.0996 | 2 | 2 | 1 | 2 |
| 2.0997 | 1 | 2 | 1 | 2 |
| 2.0998 | 2 | 2 | 1 | 2 |
| 2.0999 | 1 | 1 | 1 | 1 |
| 2.1019 | 1 | 2 | 1 | 2 |
| 2.1020 | 1 | 1 | 1 | 2 |
| 2.1059 | 3 | 8 | 3 | 8 |
| 2.1060 | 3 | 5 | 3 | 6 |
| 2.1061 | 4 | 5 | 6 | 7 |
| 2.1070 | 2 | 1 | 1 | 2 |
| 2.1071 | 2 | 1 | 1 | 2 |
| 2.1072 | 2 | 1 | 1 | 2 |
| 2.1073 | 2 | 1 | 1 | 2 |
| 2.1162 | 2 | 2 | 4 | 6 |

Beispiel 5: Nachweis der Herbizidwirkung nach dem Auflaufen der Pflanzen (Kontaktwirkung)

Eine Anzahl Unkräuter und Kulturpflanzen, sowohl monokotyle wie dikotyle, werden nach dem Auflaufen, im 4- bis 6-Blattstadium mit einer wässrigen Wirkstoffdispersion in Dosierungen von 0,5 kg/AS/ha gespritzt und dann bei 24° bis 26°C und 45-60 % relativer Luftfeuchtigkeit gehalten. 15 Tage nach der Behandlung wird der Versuch ausgewertet. Die Verbindungen 2.34, 2.38, 2.39, 2.41 und 2.42 zeigten gegen die Unkräuter Setaria italica, Lolium perenne, Solanum nigrum, Sinapis alba und Stellaria media eine sehr starke Wirkung.

Beispiel 6: Nachweis der Wuchshemmung bei tropischen Bodendeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt werden.

Patentansprüche

1. N-2-Heterocyclylphenylsulfonyl-N'-pyrimidinyl- und -triazinylharnstoffe der allgemeinen Formel I

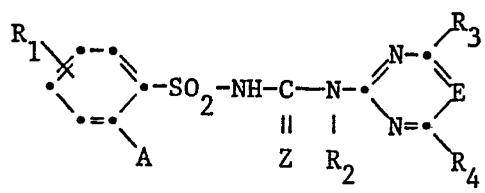

worin

Z Sauerstoff oder Schwefel,

E Stickstoff oder =CH-,

$R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R_2$ Wasserstoff, $C_1$-$C_4$-Alkyl oder $C_1$-$C_3$-Alkoxy,

$R_3$ und $R_4$ unabhängig voneinander Wasserstoff, $C_1$-$C_4$-Alkyl, $C_1$-$C_4$-Halogenalkyl, $C_3$-$C_5$-Cycloalkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy oder $-NR_5R_6$, wobei $R_5$ und $R_6$ für Wasserstoff oder $C_1$-$C_4$-Alkyl stehen, und A einen über Kohlenstoff gebundenen ungesättigten oder nur teilweise gesättigten, 5-6 gliedrigen Heterocyclus, welcher 1, 2 oder 3 Heteroatome enthält und durch $C_1$-$C_4$-Alkyl, $C_1$-$C_4$-Halogenalkyl, $C_1$-$C_4$-Alkoxy, $C_1$-$C_4$-Alkylthio, $C_2$-$C_8$-Alkoxyalkyl, Di($C_1$-$C_4$-Alkyl)amino, Halogen, Cyan oder Nitro substituiert sein kann, bedeuten, sowie die Salze dieser Verbindungen.

2. Die Verbindungen der Formel I, Anspruch 1, worin $R_1$ Wasserstoff, $R_2$ Wasserstoff oder Methyl mit der Massgabe, dass wenn A unsubstituiert oder mit $C_1$-$C_2$-Alkyl, $C_1$-$C_2$-Alkoxy, $SCH_3$, Chlor oder Brom substituiert ist, entweder $R_3$ Difluormethoxy, $C_3$-$C_5$-Cyclopropyl, Fluormethyl, Chlormethyl oder Aethoxymethyl ist und $R_4$ die im Anspruch 1 gegebene Bedeutung hat oder, dass $R_3$ Aethoxy ist und $R_4$ Aethyl, Aethoxy, Methoxyäthoxy, 2,2,2-Trifluoräthoxy oder 2-Fluoräthoxy bedeutet.

3. Die Verbindungen der Formel I, Anspruch 1, in deren A einen über Kohlenstoff gebundenen und durch $C_3$-$C_4$-Alkyl, $C_1$-$C_4$-Halogenalkyl, $C_2$-$C_4$-Alkylthio, $C_2$-$C_8$-Alkoxyalkyl, Di($C_1$-$C_4$)alkylamino, Cyan oder Nitro substituierten, ungesättigten oder nur teilweise gesättigten Heterocyclus welcher 1, 2 oder 3 Heteroatome enthält, darstellt, während E, $R_1$, $R_2$, $R_3$, $R_4$ und Z die im Anspruch 1 gegebene Bedeutung haben.

4. Die Verbindungen gemäss Anspruch 1 der Formel

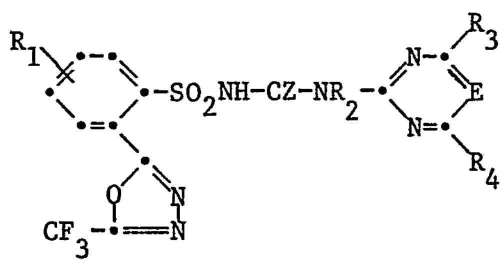

worin E, $R_1$, $R_2$, $R_3$, $R_4$ und Z die im Anspruch 1 gegebene Bedeutung haben.

5. N-[2-(5-Trilfuormethyl-1,3,4-oxadiazol-2-yl)-phenylsulfonyl]-N'-(4-methoxy-6-methylpyrimidin-2-yl)-harnstoff gemäss Anspruch 1.

6. Die Verbindungen gemäss Anspruch 1 der Formel

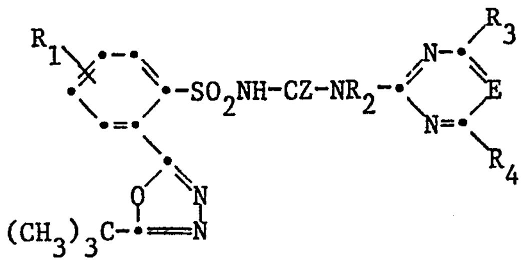

worin E, $R_1$, $R_2$, $R_3$, $R_4$ und Z die im Anspruch 1 gegebene Bedeutung haben.

7. N-[2-(5-t.Butyl-1,3,4-oxadiazol-2-yl)-phenylsulfonyl]-N'-(4'-methoxy-6-methyl-pyrimidin-2-yl)harnstoff gemäss Anspruch 1.

8. Die Verbindungen gemäss Anspruch 1 der Formel

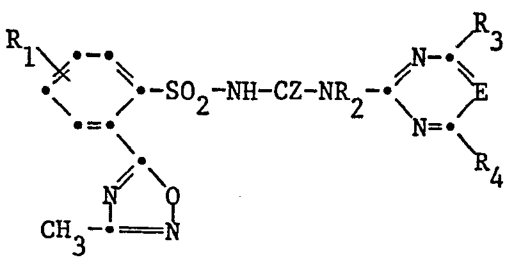

worin E, $R_1$, $R_2$, $R_3$, $R_4$ und Z die im Anspruch 2 gegebene Bedeutung haben.

9. N-[2-(3-Methyl-1,2,4-oxadiazol-5-yl)phenylsulfonyl]-N'-(4-cyclopropyl-6-methoxy-1,3,5-triazin-2-yl)harnstoff gemäss Anspruch 1.

10. N-[2-(3-Methyl-1,2,4-oxadiazol-5-yl)phenylsulfonyl]-N'-(4-methyl-6-ethyl-1,3,5-triazin-2-yl)harnstoff gemäss Anspruch 1.

11. Die Verbindungen gemäss Anspruch 1 der Formel

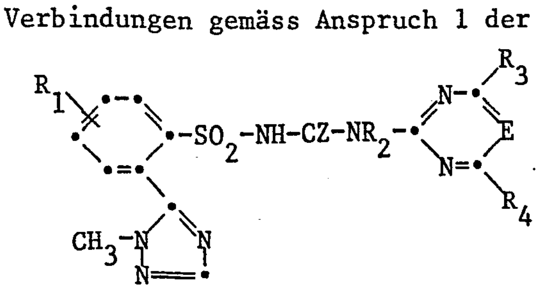

worin E, $R_1$, $R_2$, $R_3$, $R_4$ und Z die im Anspruch 2 gegebene Bedeutung haben.

12. Die Verbindungen gemäss Anspruch 2 der Formel

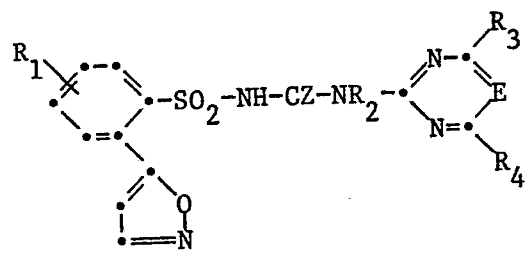

worin E, $R_1$, $R_2$, $R_3$, $R_4$ und Z die im Anspruch 2 gegebene Bedeutung haben.

13. N-[2-(1,2-Oxazol-5-yl)-phenylsulfonyl]-N'-(4-difluormethoxy-6-methyl-pyrimidin-2-yl)harnstoff gemäss Anspruch 2.

14. Die Verbindungen gemäss Anspruch 2 der Formel

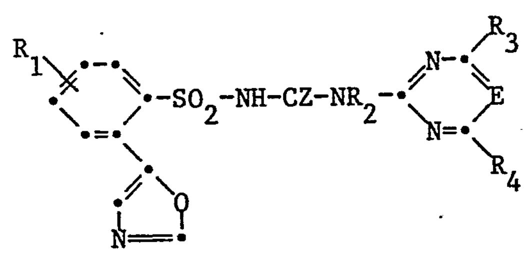

worin E, $R_1$, $R_2$, $R_3$, $R_4$ und Z die im Anspruch 2 gegebene Bedeutung haben.

15. Die Verbindungen gemäss Anspruch 2 der Formel

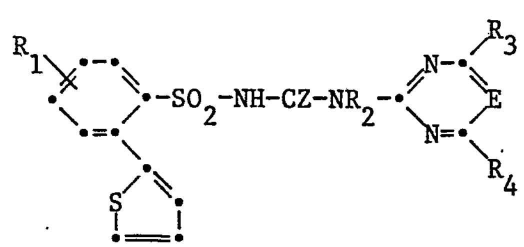

worin E, $R_1$, $R_2$, $R_3$, $R_4$ und Z die im Anspruch 2 gegebene Bedeutung haben.

16. N-[2-Thiophen-2-yl-phenylsulfonyl]-N'-(4-methoxy-6-methyl-pyrimidin-2-yl)harnstoff gemäss Anspruch 1.

17. Die Verbindungen gemäss Anspruch 1 der Formel

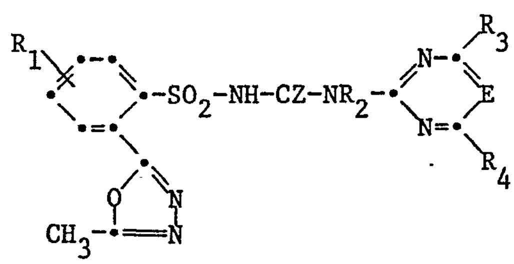

worin E, $R_1$, $R_2$, $R_3$, $R_4$ und Z die im Anspruch 2 gegebene Bedeutung haben.

18. N-[2-(5-Methyl-1,3,4-oxadiazol-2-yl)-phenylsulfonyl]-N'-(4-difluormethoxy-6-methyl-pyrimidin-2-yl)harnstoff gemäss Anspruch 2.

19. Die Verbindungen gemäss Anspruch 1 der Formel

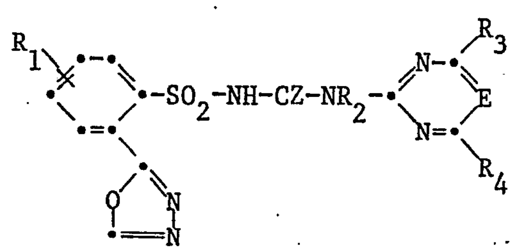

worin E, $R_1$, $R_2$, $R_3$, $R_4$ und Z die im Anspruch 2 gegebene Bedeutung haben.

20. N-[2-(1,3,4-Oxadiazol-2-yl)phenylsulfonyl]-N'-(4-difluormethoxy-6-methyl-pyrimidin-2-yl)harnstoff gemäss Anspruch 2.

21. Die Verbindungen gemäss Anspruch 1 der Formel

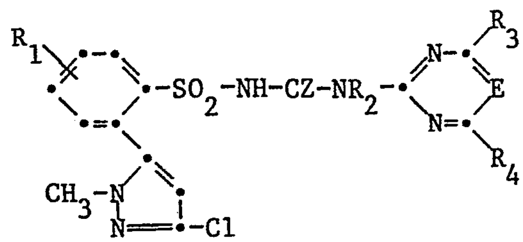

worin E, $R_1$, $R_2$, $R_3$, $R_4$ und Z die im Anspruch 2 gegebene Bedeutung haben.

22. Die Verbindungen gemäss Anspruch 1 der Formel

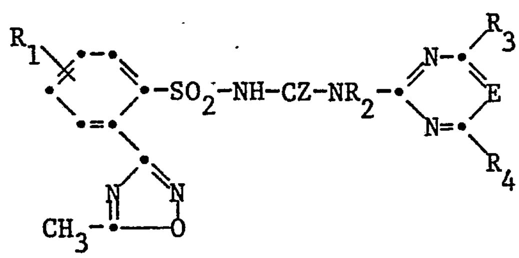

worin E, $R_1$, $R_2$, $R_3$, $R_4$ und Z die im Anspruch 1 gegebene Bedeutung haben.

23. Verfahren zur Herstellung der Verbindungen der Formel I, gemäss Anspruch 1, dadurch gekennzeichnet, dass man ein 2-Heterocyclyl-phenylsulfonamid der Formel II

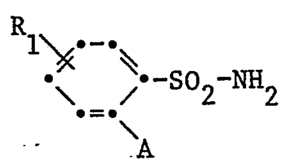

(II)

worin $R_1$ und A die unter Formel I gegebene Bedeutung haben, gegebenenfalls in Gegenwart einer Base mit einem N-Pyrimidinyl- oder -triazinylcarbamat der Formel III

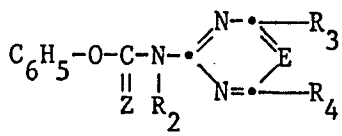

(III)

worin E, $R_2$, $R_3$, $R_4$ und Z die unter Formel I gegebene Bedeutung haben, umsetzt und gegebenenfalls in die Salze überführt.

24. Verfahren zur Herstellung der Verbindungen der Formel I gemäss Anspruch 1, dadurch gekennzeichnet, dass man ein 2-Heterocyclylphenylsulfonylisocyanat oder -isothiocyanat der Formel IV

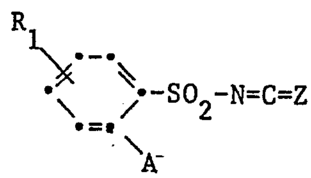

(IV)

worin A, $R_1$ und Z die unter Formel I gegebene Bedeutung haben, gegebenenfalls in Gegenwart einer Base mit einem Aminopyridin oder -triazin der Formel V

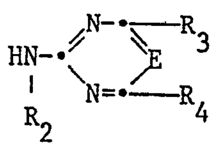

(V)

worin E, $R_2$, $R_3$ und $R_4$ die unter Formel I gegebene Bedeutung haben, umsetzt und gegebenenfalls in die Salze überführt.

25. Verfahren zur Herstellung der Verbindungen der Formel I gemäss Anspruch 1, dadurch gekennzeichnet, dass man ein N-2-Heterocyclylphenylsulfonylcarbamat der Formel VI

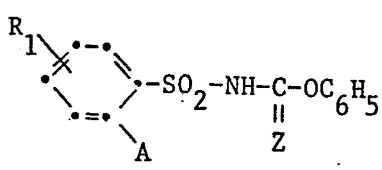

(VI)

worin A, $R_1$ und Z die unter Formel I gegebene Bedeutung haben, mit einem Aminopyrimidin oder -triazin der oben angegebenen Formel V umsetzt, und gegebenenfalls in die Salze überführt.

26. Verfahren zur Herstellung von Additionssalzen der Formel I, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

27. Ein herbizides und den Pflanzenwuchs hemmendes Mittel, dadurch gekennzeichnet, dass es neben Träger- und/oder anderen Zuschlagstoffen als Wirkstoff mindestens einen substituierten N-Phenylsulfonyl-N' pyrimidinyl- oder -triazinyl-harnstoff der Formel I enthält

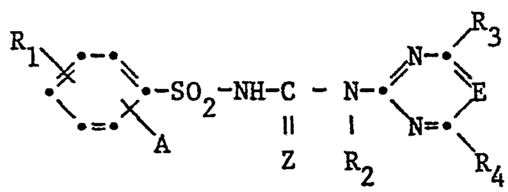

worin

Z Sauerstoff oder Schwefel,

E Stickstoff oder =CH-,

$R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxy,

$R_2$ Wasserstoff, $C_1$-$C_4$-Alkyl oder $C_1$-$C_3$-Alkoxy,

$R_3$ und $R_4$ unabhängig voneinander Wasserstoff, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy oder $-NR_5R_6$, wobei $R_5$ und $R_6$ für Wasserstoff oder Alkyl stehen, und

A einen über Kohlenstoff gebundenen, ungesättigten oder nur teilweise gesättigten 5-6 gliedrigen Heterocyclus, der 1, 2 oder 3 Hetero-

atome enthält und durch $C_1$-$C_4$-Alkyl, $C_1$-$C_4$-Halogenalkyl, $C_1$-$C_4$-Alkoxy, $C_2$-$C_8$-Alkoxyalkyl, Halogen, Cyan oder Nitro substituiert sein kann, bedeuten oder Salze dieser Verbindungen.

28. Mittel gemäss Anspruch 27, dadurch gekennzeichnet, dass es als Wirkstoff N-2-[3-Methyl-1,2,4-oxadiazol-5-yl)phenylsulfonyl-N'-(4'-(4-chlor-6-methoxypryrimidin-2-yl)harnstoff enthält.

29. Mittel gemäss Anspruch 27, dadurch gekennzeichnet, dass es als Wirkstoff N-[2-(Thiophen-2-yl)phenylsulfonyl]-N'-(4,6-dimethoxy-pyrimidin-2-yl)harnstoff enthält.

30. Mittel gemäss Anspruch 27, dadurch gekennzeichnet, dass es als Wirkstoff N-[2-(1,3,4-Oxadiazol-2-yl)phenylsulfonyl]-N'-(4-chlor-6-methoxypyrimidin-2-yl)harnstoff enthält.

31. Die Verwendung der Wirkstoffe der Formel I, gemäss Anspruch 1, oder sie enthaltender Mittel zur Bekämpfung unerwünschten Pflanzenwachstums.

32. Die Verwendung der Wirkstoffe der Formel I, gemäss Anspruch 1 oder sie enthaltender Mittel zur selektiven Unkrautbekämpfung in Nutzpflanzenkulturen.

33. Die Verwendung der Mittel gemäss Anspruch 27 zur selektiven Unkrautbekämpfung in Nutzpflanzenkulturen.

34. Verwendung gemäss Anspruch 32 in Kulturen von Getreide, Mais, Reis und Soja.

35. Verwendung gemäss Anspruch 33 in Kulturen von Getreide, Mais, Reis und Soja.

36. Die Verwendung der Wirkstoffe der Formel I, gemäss Anspruch 1, oder sie enthaltender Mittel zur Hemmung des Pflanzenwachstums.

37. Die Verwendung der Wirkstoffe der Formel I, gemäss Anspruch 1, oder sie enthaltender Mittel zur Beeinflussung des Pflanzenwachstums zum Zweck der Ertragssteigerung.

38. Die Verwendung der Wirkstoffe der Formel I, oder sie enthaltender Mittel zur selektiven pre- oder postemergenten Bekämpfung von Unkräutern in Nutzpflanzenkulturen.

39. Die Verwendung der Wirkstoffe der Formel I, oder sie enthaltender Mittel, zur Unterdrückung des Pflanzenwachstums über das 2-Blattstadium hinaus, dadurch gekennzeichnet, dass die Wirkstoffe preemergent angewendet werden.

40. Neue 2-Heterocyclylphenylsulfonylamide der Formel II

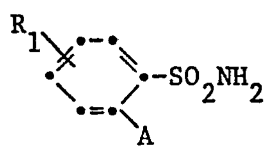

(II)

worin A und $R_1$ die im Anspruch 1 gegebene Bedeutung haben.

41. Neue 2-Heterocyclylphenylsulfonylisocyanate der Formel IV

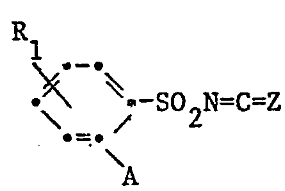

(IV)

worin A, $R_1$ und Z die im Anspruch 1 gegebene Bedeutung haben.

FO 7.3 NU/gs*/rz*

0111442

Europäisches Patentamt

# EUROPÄISCHER RECHERCHENBERICHT

Nummer der Anmeldung

EP 83 81 0511

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 030 139 (E.I. DU PONT DE NEMOURS AND COMPANY)<br>* Patentansprüche *<br>--- | 1,27 | C 07 D 413/12<br>C 07 D 409/12<br>C 07 D 403/12<br>A 01 N 47/36 |
| E | EP-A-0 083 975 (E.I. DU PONT DE NEMOURS AND COMPANY)<br>* Insgesamt *<br>--- | 1,2,3,<br>12,14,<br>17,19,<br>27,40,<br>41 | |
| E | EP-A-0 085 476 (E.I. DU PONT DE NEMOURS AND COMPANY)<br>* Insgesamt *<br>----- | 1,2,3,<br>15,27,<br>40,41 | |
| | | | **RECHERCHIERTE SACHGEBIETE (Int. Cl. 3)** |
| | | | C 07 D 413/00<br>C 07 D 409/00<br>C 07 D 403/00 |

Der vorliegende Recherchenbericht wurde für alle Patentansprüche erstellt.

| Recherchenort | Abschlußdatum der Recherche | Prüfer |
|---|---|---|
| DEN HAAG | 14-02-1984 | VAN BIJLEN H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82